(19) Europäisches Patentamt
European Patent Office
Office européen des brevets

(11) **EP 2 837 327 B1**

(12) **EUROPEAN PATENT SPECIFICATION**

(45) Date of publication and mention of the grant of the patent:
**18.10.2023 Bulletin 2023/42**

(21) Application number: **12874206.1**

(22) Date of filing: **13.04.2012**

(51) International Patent Classification (IPC):
*A61B 5/026* (2006.01)    *A61B 5/0285* (2006.01)
*G01F 1/66* (2022.01)    *G01P 5/00* (2006.01)
*G01N 21/53* (2006.01)

(52) Cooperative Patent Classification (CPC):
**G01S 17/50; A61B 5/0261; G01F 1/661;**
**G01N 21/53; G01P 5/26; G01S 17/89;** A61B 5/6866

(86) International application number:
**PCT/JP2012/060145**

(87) International publication number:
**WO 2013/153664 (17.10.2013 Gazette 2013/42)**

(54) **FLUID ASSESSMENT DEVICE AND METHOD**

FLÜSSIGKEITSBEURTEILUNGSVORRICHTUNG UND VERFAHREN

PROCÉDÉ ET DISPOSITIF D'ÉVALUATION D'UN FLUIDE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**18.02.2015 Bulletin 2015/08**

(73) Proprietor: **AIR WATER BIODESIGN INC.**
**Chuo-ku**
**Kobe-shi**
**Hyogo 650-0047 (JP)**

(72) Inventors:
• **TATEISHI, Kiyoshi**
**Kawasaki-shi**
**Kanagawa 212-0031 (JP)**

• **GOMA, Masaki**
**Kawasaki-shi**
**Kanagawa 212-0031 (JP)**

(74) Representative: **Viering, Jentschura & Partner**
**mbB**
**Patent- und Rechtsanwälte**
**Am Brauhaus 8**
**01099 Dresden (DE)**

(56) References cited:
**WO-A1-00/33053        WO-A1-2004/057313**
**WO-A1-2011/070357     WO-A1-2011/161799**
**JP-B2- 3 313 841      JP-U- S5 865 526**
**US-A- 4 590 948       US-A1- 2004 010 188**
**US-A1- 2006 115 280**

**Description**

Technical Field

The present invention relates to a fluid assessment apparatus and method which assess a fluid by detecting at least one of a flow rate and a flow velocity of the fluid such as a blood flow and so on.

Background Art

As this type of fluid assessment apparatus, for example, there is a laser blood flow meter which calculates a flow rate of a blood (for example, see a Patent Literature 1). The laser blood flow meter calculates the flow rate of the blood on the basis of a variation of a wave length of a laser light which is caused by Doppler shift, wherein the Doppler shift occurs when the laser light with which a living body is irradiated is scattered. More specifically, if the living body is irradiated with the laser light, a scattered light which is scattered by the flow of the blood in a blood vessel of the living body (namely, by movement of a blood cell which is a scattering substance) and a scattered light which is scattered by another fixed tissue (especially, a non-moving tissue such a skin tissue, a window, a transparent tube which forms a flow passage and so on) are generated. A frequency of the scattered light which is scattered by the flow of the blood changes due to laser Doppler function based on a moving velocity of the blood cell which is the scattering substance, compared to a frequency of the scattered light which is scattered by another fixed tissue. Therefore, by receiving these two types of the scattered light, a signal component which corresponds to what we call a frequency deviation (difference) signal and which is caused by a mutual interference between two types of the scattered light is obtained. The flow rate of the blood is calculated by analyzing this signal component (for example, calculating power spectrum).

Incidentally, there are additionally a Patent Literature 2 and a Patent Literature 3, as background art documents which relate to the present invention. The Patent Literature 2 discloses a fluid assessment apparatus which calculates a flow velocity of the blood and a concentration of the blood by using the light which is emitted from same light source, wherein the flow velocity of the blood indirectly represents the flow rate of the blood. The patent Literature 3 discloses a fluid assessment apparatus which calibrates the flow amount of the blood which is detected by using an ultrasonic wave Doppler signal on the basis of the flow rate of the blood which is detected by using a thermal dilution method.

US 4 590 948 A discloses that in a method and an apparatus for measuring the superficial blood flow in tissue, a section of the tissue is irradiated with monochromatic light from a laser source (cf. Abstract).

WO 2011/070357 A1 discloses that apparatus for measuring blood parameters such as chromophore, for example haemoglobin, concentration and blood flow detects light scattered from tissue surface (20) with a multispectral detector (24) that is sensitive to light across a range of different wavelengths (cf. Abstract).

WO 2011/161799 A1 discloses a photo-detection device that includes a first photoelectric conversion element and a second photoelectric conversion element that each convert input light into a current and output said current and is provided with: a photo electric converter that outputs the current difference between the current output by the first photoelectric conversion element and the current output by the second photoelectric conversion element as a detected current; and a first current-voltage converter that amplifies and converts to a voltage signal the detected current output from the photoelectric converter and outputs said voltage signal.

Citation List

Patent Literature

   Patent Literature 1: Japanese Patent No. 3313841
   Patent Literature 2: Japanese Patent Application Laid Open No. Sho62-126569
   Patent Literature 3: Japanese Examined Patent Application Publication No. Hei7-16490

Summary of Invention

Technical Problem

The fluid assessment apparatus disclosed in the Patent Literature 1 mainly calculates the flow rate of the blood which flows in the blood vessel of the living body. In this case, the concentration of the blood in the blood vessel (for example, the concentration of the blood cell such as a red blood cell and so on) hardly changes. On the other hand, the fluid assessment apparatus may detect not only the flow rate of the blood which flows in the blood vessel but also the flow rate of the blood which flows in a tube (for example, a transparent tube) which is located on an exterior of the living body and which is used during an artificial dialysis.

However, the concentration of the blood which flows in the tube which is located on the exterior of the living body and which is used during the artificial dialysis may change significantly, because of the nature of the artificial dialysis. The fluid assessment apparatus has a technical problem that the fluid assessment apparatus is not capable of calculating the flow rate of the blood appropriately (in other words, with high accuracy) when the concentration of the blood changes like this. This is why an influence of a noise which is caused by a parasitic capacitance of an input terminal of an amplifier becomes relatively large, wherein the amplifier amplifies a light receiving signal which is a result of receiving the scattered light of the laser light with which the tube is irradiated.

Incidentally, the above technical problem may occur in a same manner when not only the flow rate (or the flow velocity which indirectly represents the flow rate) of the blood but also the flow rate of an arbitrary fluid flowing in an arbitrary tube is detected.

In view of the aforementioned problem, it is therefore an object of the present invention to provide, for example, a fluid assessment apparatus and method each of which is capable of calculating at least one of the flow rate and the flow velocity of the fluid while considering the concentration of the fluid.

Solution to Problem

In order to solve the above object, the invention provides a fluid assessment apparatus according to claim 1.

In order to solve the above object, the invention provides a fluid assessment method according to claim 11.

Further embodiments of the invention are described in the dependent claims.

Brief Description of Drawings

Figures 8, 10 and 11 illustrate the claimed invention.

[FIG. 1] FIG. 1 is a block diagram illustrating a configuration of a blood flow rate calculation apparatus in a first example.
[FIG. 2] FIG. 2 is a flowchart illustrating the flow of the operation of the blood flow rate calculation apparatus in the first example.
[FIGs. 3] FIGs. 3 are graphs illustrating a correlation between the light intensity signal and the blood flow concentration in the case where the light receiving element receives the forward scattered light of the laser light and a correlation between the light intensity signal and the blood flow concentration in the case where the light receiving element receives the backward scattered light of the laser light, respectively.
[FIG. 4] FIG. 4 is a graph illustrating a power spectrum (namely, a frequency versus a power characteristics) which is obtained by performing the FFT on the sample value of the beat signal when the flow velocity of the blood is relatively fast and a power spectrum which is obtained by performing the FFT on the sample value of the beat signal when the flow velocity of the blood is relatively slow.
[FIG. 5] FIGs. 5 are graphs illustrating both of an ideal value and an actual value of each of the power spectrum which is obtained by performing the FFT on the sample value of the beat signal when the blood flow concentration is relatively large and the power spectrum which is obtained by performing the FFT on the sample value of the beat signal when the blood flow concentration is relatively small.
[FIG. 6] FIG. 6 is a graph illustrating a correlation between the flow velocity of the blood and an average frequency of the power spectrum for each blood flow concentration.
[FIG. 7] FIG. 7 is a graph illustrating a relationship between the blood flow concentration and each of a slope correction value and an intercept correction value, wherein the slope correction value and the intercept correction value are used to calculate the flow velocity of the blood from the average frequency of the power spectrum.
[FIG. 8] FIG. 8 is a block diagram illustrating a configuration of a blood flow rate calculation apparatus in a second example.
[FIG. 9] FIG. 9 is a block diagram illustrating a configuration of a blood flow rate calculation apparatus in a third example.
[FIG. 10] FIG. 10 is a block diagram illustrating a configuration of a blood flow rate calculation apparatus in a fourth example.
[FIG. 11] FIG. 11 is a block diagram illustrating a configuration of a specific example of the light receiving element and the amplifier which each of the blood flow rate calculation apparatus in the first example to the blood flow rate calculation apparatus in the fourth example is provided with

Description of Embodiment

Hereinafter, as an embodiment of the present invention, an embodiment of each of a fluid assessment apparatus and method will be described.

(Embodiment of Fluid Assessment Apparatus)

<1> A fluid assessment apparatus of a present embodiment is provided with: an irradiating device which is configured to irradiate measurement target with at least a laser light, wherein a fluid flows in an interior of the measurement target; a light receiving device which is configured to receive at least the laser light with which the measurement target is irradiated; a first calculating device which is configured to calculate a concentration of the fluid from a first received light signal based on a signal intensity of a received light signal which the light receiving device receives; and a second calculating device which is configured to calculate at least one of a flow rate of the fluid and a flow velocity of the fluid from (i) a second received light signal based on a variation of a frequency of the received light signal which is caused by a Doppler shift of the laser light with which the measurement target is irradiated and (ii) the concentration which the first calculating device calculates. The light receiving device includes a first photo diode and a second photo diode each of which is configured to receive the laser light and convert the laser light to electrical current, the first and second photo diodes being coupled in series such that same polarity nodes of the first and second photo diodes are coupled with each other, the light receiving device being configured to output, as the second received light signal, differential electrical current between the electrical current outputted from the first photo diode and the electrical current outputted from the second photo diode. The second calculating device includes an amplifier that is configured to amplify and convert the second light received light signal into voltage signal and output the voltage signal. The signal intensity is a signal intensity of a signal component of the received light signal whose frequency is relatively low and the variation of the frequency is a variation of a frequency of a signal component of the received light signal whose frequency is relatively high. The light receiving device includes: (i) a first light receiving device which obtains the first received light signal by receiving the laser light with which the measurement target is irradiated and (ii) a second light receiving device which obtains the second received light signal by receiving the laser light with which the measurement target is irradiated. A light receiving area of the first light receiving device is larger than a light receiving area of the second light receiving device.

According to the fluid assessment apparatus of the present embodiment, the irradiating device irradiates the measurement target with the laser light. The fluid flows in the interior of the measurement target. It is preferable that the fluid be irradiated with the laser light. Incidentally, a living body such as a human, an animal and the like and a blood are listed as one examples of the measurement target and the fluid, respectively. Alternatively, an artificial tube in which the blood flows and the blood are listed as another examples of the measurement target and the fluid, respectively. Alternatively, tube having a window through which the light is capable of penetrating (going, being transmitted) and a transparent tube and a fluid (for example, a fluid which includes, as a component, a scattering substance which scatters the light such as an ink, an oil, a sewage water, a seasoning (a condiment) and the like) flowing in the tube are listed as another examples of the measurement target and the fluid, respectively.

The light receiving device receives the laser light with which the measurement target is irradiated by the irradiating device. Specifically, for example, the light receiving device receives the laser light (what we call, a scattered light) which is scattered by the measurement target. The light receiving device may receive the laser light (what we call, a forward scattered light which corresponds to a transmitted light) which penetrates (goes, is transmitted) through the measurement target, and may receive the laser light (what we call, a backward scattered light which corresponds to a reflected light) which is reflected by the measurement target. As a result, the light receiving device outputs the received light signal based on the received laser light (especially, the scattered light of the received laser light).

The first calculating device calculates the concentration of the fluid from the first received light signal based on the signal intensity of the received light signal (for example, based on an average of the signal intensity, a signal intensity of a DC (Direct Current) component included in the received light signal, and the like), in other words, from the first received light signal which corresponds to a signal component representing the signal intensity of the received light signal. For example, in the case where the light receiving device receives the laser light (namely, the forward scattered light) which penetrates through the measurement target, the larger the concentration of the fluid is (namely, the more the fluid includes the scattering substance and the like which prevents the penetration (transmission) of the laser light), the smaller the signal intensity of the received light signal (for example, an amplitude or a peak value of the first received light signal) is. Alternatively, for example, in the case where the light receiving device receives the laser light (namely, the backward scattered light) which is reflected by the measurement target, the larger the concentration of the fluid is (namely, the more the fluid includes the scattering substance and the like which reflects the laser light), the larger the signal intensity of the received light signal is. The first calculating device calculates the concentration by using the above described relationship between the signal intensity of the received light signal and the concentration.

Incidentally, the "concentration of the fluid" in the present embodiment corresponds to a concentration of the scattering substance included in the fluid, because the concentration is calculated by using the above described relationship between the signal intensity of the received light signal and the concentration.

The second calculating device calculates at least one of the flow rate and the flow velocity from the second received light signal based on the variation of the frequency of the received light signal which is caused by the Doppler shift of the laser light (especially, the scattered light of the laser light) with which the measurement target is irradiate, in other words, from the second received light signal which corresponds to including the variation of the frequency of the received light signal. Specifically, for example, when the measurement target is irradiated with the laser light, the scattered light which is caused by the flow of the fluid in the interior of the measurement target arises. The scattered light includes: a scattered light which is scattered by the scattering substance (especially, the scattering substance which is moving) included in the fluid; and a scattered light which is scattered by another fixed tissue (especially, a non-moving tissues such as a skin tissue, a window, a transparent tube which forms a flow passage and so on). A frequency of the scattered light which is scattered by scattering substance which is moving changes due to a laser Doppler function based on the flow velocity of the fluid, compared to a frequency of the scattered light which is scattered by another fixed tissue. The received light signal includes a signal component which corresponds to what we call a frequency deviation (difference) signal, due to a mutual interference between two types of the scattered light. At least one of the flow rate and the flow velocity is calculated by analyzing this signal component.

Especially in the present embodiment, the second calculating device calculates at least one of the flow rate and the flow velocity on the basis of the concentration which the first calculating device calculates, in addition to the second received light signal based on the variation of the frequency of the received light signal which is caused by the Doppler shift of the laser light. For example, the second calculating device conventionally obtains same flow rate (or same flow velocity) as a result of the calculation if the variation of the frequency of the received light signal is same. However, in the present embodiment, even if the variation of the frequency of the received light signal is same, the second calculating device obtains different flow rate (or different flow velocity) as a result of the calculation if the concentration is different. For example, it is assumed that the second calculating device obtains a first flow rate (or a first flow velocity) as a result of the calculation if the variation of the frequency is a predetermined aspect and the concentration is a first concentration. In this case, if the variation of the frequency is the predetermined aspect and the concentration is a second concentration which is different from the first concentration, the second calculating device may obtain a second flow rate which is different from the first flow rate (or a second flow velocity which is different from the first flow velocity) as a result of the calculation.

If at least one of the flow rate and the flow velocity is calculated without considering the concentration which the first calculating device calculates, at least one of the flow rate and the flow velocity may not be calculated appropriately. The reason is as follows. If the concentration of the fluid is relatively small, the number of the scattering substance, which moves in the fluid, per unit volume is small and thus a power of a light beat signal decreases, wherein the light beat signal is included in the received light signal and is caused by the Doppler shift of the laser light which is due to the moving of the scattering substance. On the other hand, a power of a noise which is caused by a parasitic capacitance of an input terminal of an amplifier does not change, wherein the amplifier amplifies the received light signal which is a result of receiving the scattered light of the laser light with which the measurement target is irradiated, and thus an influence of the noise becomes relatively large. Namely, if the concentration of the fluid is relatively small, the variation of the frequency of the received light signal which is a result of receiving the scattered light of the laser light with which the measurement target is irradiated may be different from an originally appeared variation of the frequency, due to the relatively large influence of the noise which is caused by the parasitic capacitance of the input terminal of the amplifier. Incidentally, even if the concentration of the fluid is relatively large, same technical problem may possibly arise. Therefore, the variation of the frequency of the received light signal which is a result of receiving the scattered light of the laser light with which the measurement target is irradiated may undesirably changes from an originally appeared variation of the frequency due to the concentration of the fluid. However, the fluid assessment apparatus of the present embodiment is capable of calculating at least one of the flow rate and the flow velocity appropriately, because the fluid assessment apparatus of the present embodiment refers to the concentration of the fluid in order to prevent the influence of the noise.

As described above, the fluid assessment apparatus of the present embodiment is capable of calculating at least one of the flow rate and the flow velocity appropriately (in other words, with high accuracy) while considering the concentration of the fluid. Especially, even when the blood (namely, the blood whose concentration (for example, the concentration of the red blood cell) changes relatively easily), which flows in the tube (for example, the transparent tube) which is located on the exterior of the living body and which is used during an artificial dialysis, is one example of the fluid, the fluid assessment apparatus of the present embodiment is capable of calculating at least one of the flow rate and the flow velocity of the fluid (namely, the blood) appropriately. Of course, even when the blood (namely, the blood whose concentration hardly changes), which flows in a blood vessel of the living body, is one example of the fluid, the fluid assessment apparatus of the present embodiment is capable of calculating at least one of the flow rate and the flow velocity of the fluid (namely, the blood) appropriately.

Incidentally, there is a positive correlation between the flow rate of the fluid and the flow velocity of the fluid under such a condition that a flow passage in which the fluid flows does not change. Namely, the larger the flow rate of the fluid which flows in a certain flow passage is, the larger the flow velocity of the fluid which flows in the certain flow passage is. On the other hand, the smaller the flow rate of the fluid which flows in the certain flow passage is, the smaller the flow velocity of the fluid which flows in the certain flow passage is. Therefore, the calculation of the flow rate is substantially same as the calculation of the flow velocity. Namely, the calculation of the flow velocity substantially corresponds to the indirect calculation of the flow rate and the calculation of the flow rate substantially corresponds to the indirect calculation of the flow velocity.

<2> In another aspect of the fluid assessment apparatus of the present embodiment, the irradiating device includes: (i) a first irradiating device which irradiates the measurement target with a measurement light which is a light whose type is different from the type of the laser light; and (ii) a second irradiating device which irradiates the measurement target with the laser light, the light receiving device (i) obtains the first received light signal by receiving the measurement light with which the measurement target is irradiated and (ii) obtains the second received light signal by receiving the laser light with which the measurement target is irradiated, the first calculating device calculates the concentration from the first received light signal, the second calculating device calculates at least one of the flow rate and the flow velocity from (i) the second received light signal and (ii) the concentration which the first calculating device calculates

According to this aspect, the irradiating device is divided into the first irradiating device which is mainly used to calculate the concentration and the second irradiating device which is mainly used to calculate at least one of the flow rate and the flow velocity. Therefore, the first irradiating device is capable of irradiating the measurement light (for example, a light whose wavelength is 805 nanometer which is not affected by a degree of oxygen saturation in the blood flow which is one example of the fluid) which is suitable for the calculation of the concentration without considering the calculation of at least one of the flow rate and the flow velocity much. On the other hand, the second irradiating device is capable of irradiating the laser light (for example, a light whose wavelength is 780 nanometer which has a high monochromatic characteristics and a high coherency and which can be emitted from a semiconductor laser that is used widely) which is suitable for the calculation of at least one of the flow rate and the flow velocity without considering the calculation of the concentration much.

<3> In the fluid assessment apparatus of the present embodiment, the light receiving device includes: (i) a first light receiving device which obtains the first received light signal by receiving the laser light with which the measurement target is irradiated and (ii) a second light receiving device which obtains the second received light signal by receiving the laser light with which the measurement target is irradiated, the first calculating device calculates the concentration from the first received light signal, the second calculating device calculates at least one of the flow rate and the flow velocity from (i) the second received light signal and (ii) the concentration which the first calculating device calculates

According to this aspect, the light receiving device is divided into the first light receiving device which is mainly used to calculate the concentration and the second light receiving device which is mainly used to calculate at least one of the flow rate and the flow velocity.

<4> In another aspect of the fluid assessment apparatus of the present embodiment, as described above, the fluid assessment apparatus is provided with the first irradiation device and the second irradiation device. Furthermore, the light receiving device includes: (i) a first light receiving device which obtains the first received light signal by receiving the measurement light with which the measurement target is irradiated and (ii) a second light receiving device which obtains the second received light signal by receiving the laser light with which the measurement target is irradiated, the first calculating device calculates the concentration from the first received light signal, the second calculating device calculates at least one of the flow rate and the flow velocity from (i) the second received light signal and (ii) the concentration which the first calculating device calculates.

According to this aspect, the irradiating device is divided into the first irradiating device which is mainly used to calculate the concentration and the second irradiating device which is mainly used to calculate at least one of the flow rate and the flow velocity. Moreover, the light receiving device is divided into the first light receiving device which is mainly used to calculate the concentration and the second light receiving device which is mainly used to calculate at least one of the flow rate and the flow velocity.

<5> In the fluid assessment apparatus which is provided with the first light receiving device and the second light receiving device, a light receiving area of the first light receiving device is larger than a light receiving area of the second light receiving device.

According to this aspect, the light receiving area of the first receiving device which is mainly used to calculate the concentration is relatively large. Thus, it is possible to improve a S/N ratio of the first received light signal which is used to detect the concentration. On the other hand, the light receiving area of the second receiving device which is mainly used to calculate at least one of the flow rate and the flow velocity is relatively small. Thus, it is possible to reduce the influence of the noise of the amplifier which contributes a decrease of the accuracy of calculating at least one of the flow rate and the flow velocity (more specifically, to reduce the parasitic capacitance of the input terminal of the amplifier which contributes the generation of the noise).

Incidentally, a distance between the irradiating device and the second light receiving device whose light receiving area is relatively small may be shorter than a distance between the irradiating device and the first light receiving device whose light receiving area is relatively large such that both of the first light receiving device and the second light receiving device are capable of receiving at least one of the measurement light and the laser light appropriately. In other words, it is preferable that the second light receiving device whose light receiving area is relatively small be as close as possible to the irradiating device.

<6> In another aspect of the fluid assessment apparatus of the present embodiment, the second calculating device calculates at least one of the flow rate and the flow velocity on the basis of correlation information, wherein the correlation information defines a correlation between the variation of the frequency and at least one of the flow rate and the flow velocity for each concentration.

According to this aspect, the second calculating device is capable of calculating at least one of the flow rate and the flow velocity, relatively easily, on the basis of the correlation information. Specifically, the second calculating device is capable of calculating at least one of the flow rate and the flow velocity from the variation of the frequency, by referring to the correlation which corresponds to the concentration which the first calculating device calculates.

<7> In another aspect of the fluid assessment apparatus of the present embodiment, the second calculating device calculates at least one of the flow rate and the flow velocity on the basis of correlation information, wherein the correlation information defines a correlation between an average frequency which can be obtained by analyzing the variation of the frequency and at least one of the flow rate and the flow velocity for each concentration.

According to this aspect, the second calculating device is capable of calculating at least one of the flow rate and the flow velocity, relatively easily, on the basis of the correlation information. Specifically, the second calculating device is capable of calculating at least one of the flow rate and the flow velocity from the average frequency which can be obtained by analyzing the variation of the frequency, by referring to the correlation which corresponds to the concentration which the first calculating device calculates.

Incidentally, the second calculating device may calculate at least one of the flow rate and the flow velocity on the basis of correlation information, wherein the correlation information defines a correlation between an arbitrary parameter which can be obtained by analyzing the variation of the frequency and at least one of the flow rate and the flow velocity for each concentration.

<8> In an another aspect of the fluid assessment apparatus which calculates at least one of the flow rate and the flow velocity on the basis of the correlation information which defines the correlation between the average frequency and at least one of the flow rate and the flow velocity for each concentration, the correlation information defines the correlation between the average frequency and at least one of the flow rate and the flow velocity for each concentration such that at least one of the flow rate and the flow velocity which is calculated when the concentration is relatively small is smaller than at least one of the flow rate and the flow velocity which is calculated when the concentration is relatively large under such a condition that the average frequency does not change.

According to this aspect, the second calculating device is capable of calculating at least one of the flow rate and the flow velocity, relatively easily, on the basis of the correlation information. Specifically, an example in which the average frequency which is obtained by analyzing the variation of the frequency is a first frequency will be explained. In this example, if the concentration is a relatively small first concentration, the second calculating device obtains at least one of a first flow rate which is a relatively small flow rate and a first flow velocity which is a relatively small flow velocity as a result of the calculation. On the other hand, if the concentration is a relatively large second concentration, the second calculating device obtains at least one of a second flow rate which is a relatively large flow rate and a second flow velocity which is a relatively large flow velocity as a result of the calculation.

Incidentally, one can argue that the correlation information in this aspect defines the correlation between the average frequency and at least one of the flow rate and the flow velocity for each concentration such that the average frequency in the case where the concentration is relatively small is higher than the average frequency in the case where the concentration is relatively large under such a condition that at least one of the flow rate and the flow velocity does not change.

Moreover, as described above, the second calculating device may calculate at least one of the flow rate and the flow velocity on the basis of the correlation information, wherein the correlation information defines the correlation between the arbitrary parameter which can be obtained by analyzing the variation of the frequency and at least one of the flow rate and the flow velocity for each concentration. In this aspect, the correlation information may define the correlation between the arbitrary parameter and at least one of the flow rate and the flow velocity for each concentration such that at least one of the flow rate and the flow velocity which is calculated when the concentration is relatively small is smaller than at least one of the flow rate and the flow velocity which is calculated when the concentration is relatively large under such a condition that the arbitrary parameter does not change.

<9> In the fluid assessment apparatus of the present embodiment, a cycle with which the second calculating device calculates at least one of the flow rate and the flow velocity is shorter than a cycle with which the first calculating device calculates the concentration.

The first calculating device is capable of calculating the concentration with a relatively long cycle (in other words, with a relatively low frequency (repetition)) on the basis of the signal intensity of the received light signal which includes a relatively low frequency (in other words, on the basis of the signal intensity which is a signal component whose frequency is relatively low). Moreover, the second calculating device is capable of calculating at least one of the flow rate and the flow velocity with a relatively short cycle (in other words, with a relatively high frequency (repetition)) on the basis of the variation of the frequency of the received light signal which includes a relatively high frequency (in other words, on the basis of the variation of the frequency which is a signal component whose frequency is relatively high).

<10> In another aspect of the fluid assessment apparatus of the present embodiment, the fluid is a blood flow of a living body, wherein the living body is the measurement target.

According to this aspect, the first calculating device is capable of calculating the concentration of the blood (for example, the concentration of the red blood cell and so on which is the scattering substance). Moreover, the second calculating device is capable of calculating at least one of the flow rate of the blood (namely, a blood flow rate) and the flow velocity of the blood.

(Embodiment of Fluid Assessment Method)

<11> A fluid assessment method of the present embodiment is a fluid assessment method in a fluid assessment apparatus, the fluid assessment apparatus is provided with: an irradiating device which is configured to irradiate measurement target with at least a laser light, wherein a fluid flows in an interior of the measurement target; and a light receiving device which is configured to receive at least the laser light with which the measurement target is irradiated, the fluid assessment method is provided with: a first calculating process which calculates a concentration of the fluid from a first received light signal based on a signal intensity of a received light signal which the light receiving device receives; and a second calculating process which calculates at least one of a flow rate of the fluid and a flow velocity of the fluid from (i) a second received light signal based on a variation of a frequency of the received light signal which is caused by a Doppler shift of the laser light with which the measurement target is irradiated and (ii) the concentration which the first calculating process calculates. The light receiving device including a first photo diode and a second photo diode each of which is configured to receive the laser light and convert the laser light to electrical current. The first and second photo diodes are coupled in series such that same polarity nodes of the first and second photo diodes are coupled with each other. The light receiving device is configured to output, as the second received light signal, differential electrical current between the electrical current outputted from the first photo diode and the electrical current outputted from the second photo diode. The second calculating process amplifies and converts the second light received light signal into voltage signal and outputs the voltage signal. The signal intensity is a signal intensity of a signal component of the received light signal whose frequency is relatively low. The variation of the frequency is a variation of a frequency of a signal component of the received light signal whose frequency is relatively high. The light receiving device includes: (i) a first light receiving device which obtains the first received light signal by receiving the laser light with which the measurement target is irradiated and (ii) a second light receiving device which obtains the second received light signal by receiving the laser light with which the measurement target is irradiated. A light receiving area of the first light receiving device is larger than a light receiving area of the second light receiving device.

According to the fluid assessment method of the present embodiment, it is possible to enjoy various effects which the above described fluid assessment apparatus of the present embodiment enjoys.

Incidentally, the fluid assessment method of the present embodiment may adopt various aspects in accordance with the various aspect which the fluid assessment apparatus of the present embodiment adopts.

These operations and other advantages of the present embodiment will become more apparent from the examples explained below.

As described above, the fluid assessment apparatus of the present embodiment is provided with the irradiating device, the light receiving device, the first calculating device and the second calculating device. The fluid assessment method of the present embodiment is provided with the first calculating process and the second calculating process. Therefore, at least one of the flow rate of the fluid and the flow velocity of the fluid is calculated appropriately while the concentration of the fluid is considered.

Examples

Hereinafter, examples of the fluid assessment apparatus will be described with reference to the drawings. Incidentally, hereinafter, an examples in which the fluid assessment apparatus is applied to a blood flow rate calculation apparatus which detects a flow rate of a blood (namely, a blood flow rate) flowing in a tube 100, wherein the tube 100 constitutes a blood flow circuit of an artificial dialysis apparatus. However, the fluid assessment apparatus may be applied to an arbitrary flow rate detection apparatus which detects the flow rate of the blood (namely, the blood flow rate) flowing in a blood vessel of a living body and the flow rate of an arbitrary liquid (for example, an ink, an oil, a sewage water, seasoning (a condiment) and the like) other than the blood.

(1) First Example

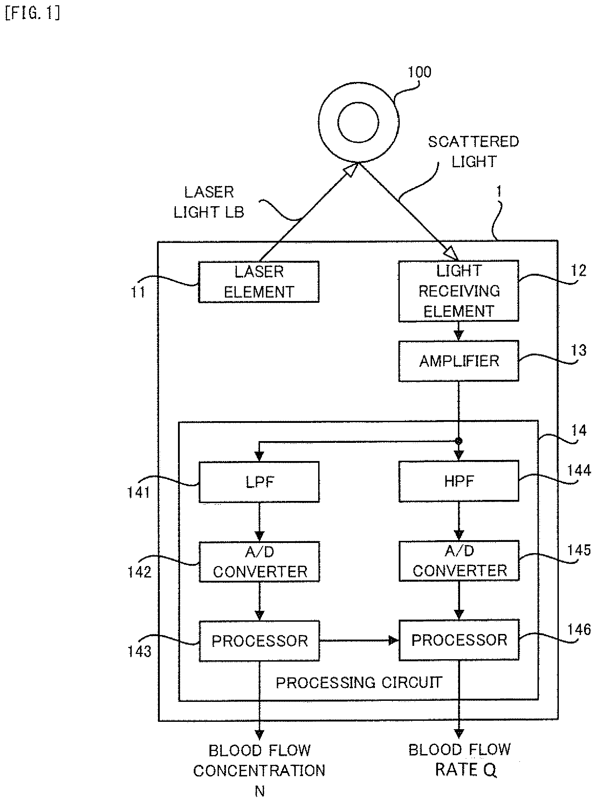

Firstly, with reference to FIG. 1 to FIG. 7, a blood flow rate calculation apparatus 1 in a first example will be explained.

(1-1) Configuration of Blood Flow rate Calculation Apparatus

Firstly, with reference to FIG. 1, a configuration of the blood flow rate calculation apparatus 1 in the first example will be explained. FIG. 1 is a block diagram illustrating the configuration of the blood flow rate calculation apparatus 1 in the first example.

As illustrated in FIG. 1, the blood flow rate calculation apparatus 1 in the first example is provided with: a laser element 11; a light receiving element 12; an amplifier 13; and a processing circuit 14.

The laser element 11 constitutes one example of the "irradiating device" and irradiates the tube 100 with a laser light LB. It is preferable that the laser element 11 irradiate a flow passage (namely, a flow passage in which the blood flows) in the tube 100 with the laser light LB.

The light receiving element 12 constitutes one example of the "light receiving device" and receives a scattered light of the laser light LB with which the tube 100 is irradiated. The scattered light which the light receiving element 12 receives includes a scattered light which is scattered by the blood (especially, a blood cell which is a moving scattering substance and which is included in the blood) flowing in the tube 100 and a scattered light which is scattered by a static tissue (for example, the tube 100 itself). The light receiving element 12 generates the received light signal which is obtained by converting the received scattered light into an electric signal.

The amplifier 13 converts the received light signal which is an electric current signal outputted from the light receiving element 12 into a voltage signal and amplifies the voltage signal.

The processing circuit 14 calculates the flow amount (namely, the blood flow rate) Q of the blood flowing in the tube 100 and a concentration (namely, a blood flow concentration) N of the blood. In order to calculates the blood flow rate Q and the blood flow concentration N, the processing circuit 14 is provided with: a LPF (Low Pass Filter) 141; an A/D converter 142; a processor 143; a HPF (High Pass Filter) 144; an A/D converter 145; and a processor 146.

The LPF 141 cuts a signal component in the other frequency band other than a low frequency signal component from a signal component included in the output of the amplifier 13 (namely, in the received light signal corresponding to the scattered light which the light receiving element 12 receives). As a result, the LPF 141 outputs a light intensity signal which corresponds to the low frequency signal component in the signal component included in the output of the amplifier 13 into the A/D converter 142. Incidentally, the light intensity signal is a signal which directly or indirectly represents a signal intensity (for example, an average light intensity) of the scattered light which the light receiving element 12 receives.

Incidentally, the light intensity signal is often a signal component whose frequency is equal to or less than 1 kHz, for example. Therefore, it is preferable that the LPF 141 have a cut-off frequency which is capable of allowing the light intensity signal to pass and cutting a signal component whose frequency is higher than the frequency of the light intensity signal (for example, is equal to or higher than 1 kHz).

The A/D converter 142 performs an A/D converting process (namely, a quantizing process) on the light intensity signal outputted from the LPF 141. As a result, the A/D converter 142 outputs sample value of the light intensity signal (namely, the quantized light intensity signal) included in the voltage signal corresponding to the scattered light which the light receiving element 12 receives into the processor 143.

The processor 143 constitutes one example of the "first calculating device" and calculates the blood flow concentration N on the basis of the output of the A/D converter 142 (namely, the sample value of the light intensity signal included in the voltage signal corresponding to the scattered light which the light receiving element 12 receives).

The HPF 144 cuts a signal component in the other frequency band other than a high frequency signal component from a signal component included in the output of the amplifier 13 (namely, in the received light signal corresponding to the scattered light which the light receiving element 12 receives). As a result, the HPF 144 outputs a beat signal which corresponds to the high frequency signal component in the signal component included in the output of the amplifier 13 into the A/D converter 142. Incidentally, the beat signal which the HPF 144 extracts is a beat signal which is generated by a mutual interference between the scattered light which is scattered by the blood cell which is a moving scattering substance and the scattered light which is scattered by the static tissue.

Incidentally, the beat signal is often a signal component whose frequency is equal to or more than 1 kHz, for example. Therefore, it is preferable that the HPF 144 have a cut-off frequency which is capable of allowing the beat signal to pass and cutting a signal component whose frequency is lower than the frequency of the beat signal (for example, is equal to or lower than 1 kHz). However, a BPF (Band Pass Filter) which is capable of allowing the beat signal to pass and cutting both of a signal component whose frequency is lower than the frequency of the beat signal and a signal component whose frequency is higher than the frequency of the beat signal may be used instead of the HPF 144.

The A/D converter 145 performs an A/D converting process (namely, a quantizing process) on the beat signal outputted from the HPF 144. As a result, the A/D converter 145 outputs sample value of the beat signal (namely, the quantized beat signal) included in the voltage signal corresponding to the scattered light which the light receiving element 12 receives into the processor 146.

The processor 146 constitutes one example of the "second calculating device" and performs a frequency analysis on the output of the A/D converter 145 (namely, the sample value of the beat signal included in the voltage signal corresponding to the scattered light which the light receiving element 12 receives) by using a FFT (Fast Fourier Transform). As a result, the processor 146 calculates the blood flow rate Q. Especially, in the first example, as described later in detail, the processor 146 calculates the blood flow rate Q on the basis of not only the result of the frequency analysis of the beat signal by using the FFT which is performed on the output of the A/D converter 145 but also the blood flow concentration N which the processor 143 calculates.

Incidentally, the blood flow concentration N which the processor 143 calculates and the blood flow rate Q which the processor 146 calculates may be outputted into the exterior of the blood flow rate calculation apparatus 1 (or into a non-illustrated processing block which the blood flow rate calculation apparatus 1 is provided with therein) in an appropriate timing.

(1-2) Operation of Blood Flow rate Calculation Apparatus

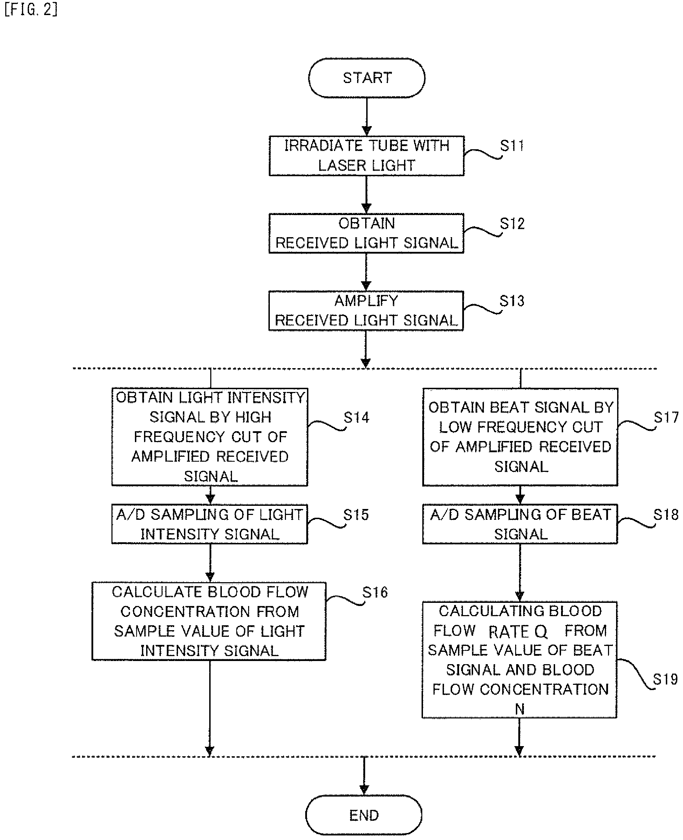

Next, with reference to FIG. 2, a flow of the operation of the blood flow rate calculation apparatus 1 in the first example will be explained. FIG. 2 is a flowchart illustrating the flow of the operation of the blood flow rate calculation apparatus 1 in the first example.

As illustrated in FIG. 2, the laser element 11 irradiates the tube 100 with the laser light LB (step S11). It is preferable that the laser element 11 irradiate the flow passage (namely, the flow passage in which the blood flows) in the tube 100 with the laser light LB.

Then, the light receiving element 12 receives the scattered light of the laser light LB from the tube 100 (step S12). More specifically, the light receiving element 12 receives the scattered light which is scattered by the blood (especially, the blood cell which is the moving scattering substance and which is included in the blood) flowing in the tube 100 and the scattered light which is scattered by the static tissue (for example, the tube 100 itself). Incidentally, a forward scattered light which corresponds to a transmitted light of the laser light LB with which the tube 100 is irradiated may be used as the scattered light. A backward scattered light which corresponds to a reflected light of the laser light LB with which the tube 100 is irradiated may be used as the scattered light.

Then, the light receiving element 12 generates the received light signal which is obtained by converting the received scattered light into an electric signal (step S12). Then, the light receiving element 12 outputs the generated received light signal into the amplifier 13.

The received light signal includes a signal component corresponding to a light intensity of the scattered light (namely, the above described light intensity signal, and a signal component whose frequency is relatively low). Moreover, the received light signal includes a signal component corresponding to a beat signal light (namely, the above described beat signal, and a signal component whose frequency is relatively high) which is generated by the mutual interference between the scattered light which is scattered by the blood cell which is a moving scattering substance and the scattered light which is scattered by the static tissue. Here, a frequency of the scattered light which is scattered by the blood cell which is a moving scattering substance changes due to a laser Doppler function based on a flow velocity v of the blood, compared to a frequency of the scattered light which is scattered by the static tissue. Therefore, the signal component corresponding to the beat signal light (namely, the beat signal) includes a signal component representing a variation of the frequency based on the flow velocity v of the blood.

Then, the amplifier 13 converts the received light signal which is outputted from the light receiving element 12 (namely, the received light signal corresponding to the scattered light which the light receiving element 12 receives) into the voltage signal and amplifies the voltage signal (step S13). Then, the amplifier 13 outputs the amplified received light signal into the processing circuit 14. More specifically, the amplifier 13 outputs the amplified received light signal into both of the LPF 141 and the HPF 144.

Then, the LPF 141 cuts the signal component in the other frequency band other than the low frequency signal component from the signal component included in the output of the amplifier 13. As a result, the LPF 141 obtains the light intensity signal which corresponds to the low frequency signal component in the signal component included in the output of the amplifier 13 (step S14). Then, the LPF 141 outputs the obtained light intensity signal into the A/D converter 142.

Then, the A/D converter 142 performs the A/D converting process (namely, the quantizing process) on the light intensity signal outputted from the LPF 141 (step S15). Specifically, if a sampling cycle of the A/D converter 142 is Ta1, the A/D converter 142 outputs the sample value of the light intensity signal (namely, the quantized light intensity signal) every cycle Ta1. Then, the A/D converter 142 outputs the sample value of the light intensity signal (namely, the quantized light intensity signal) corresponding to the light intensity of the scattered light which the light receiving element 12 receives into the processor 143.

Then, the processor 143 calculates the blood flow concentration N on the basis of the output of the A/D converter 142 (namely, the sample value of the light intensity signal corresponding to the light intensity of the scattered light which the light receiving element 12 receives) (step S16).

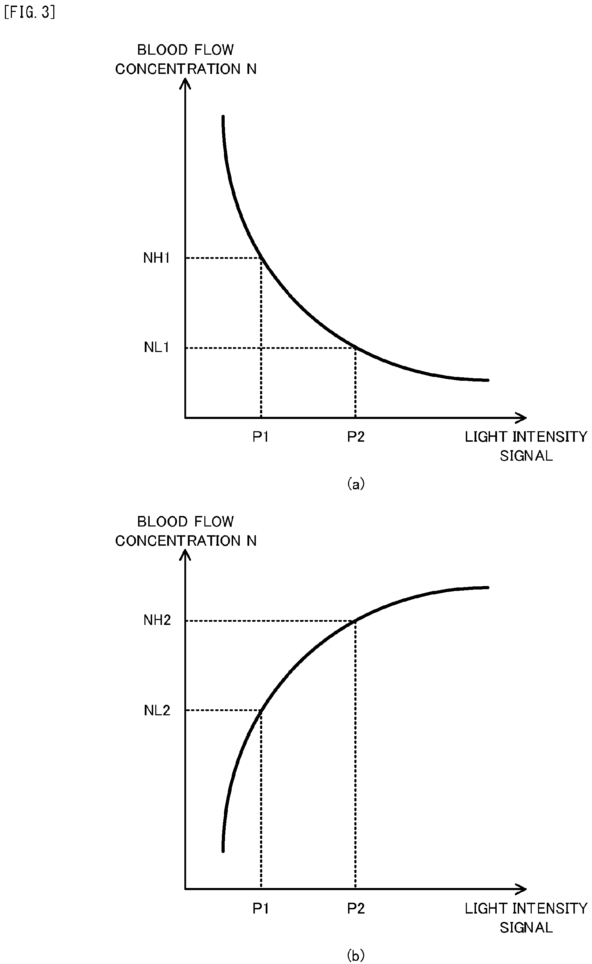

Here, with reference to FIGs. 3, an operation of calculating the blood flow concentration N on the basis of the light intensity signal will be explained. FIGs. 3 are graphs illustrating a correlation between the light intensity signal and the blood flow concentration N in the case where the light receiving element 12 receives the forward scattered light of the laser light LB and a correlation between the light intensity signal and the blood flow concentration N in the case where the light receiving element 12 receives the backward scattered light of the laser light LB, respectively.

FIG. 3(a) illustrates the correlation between the light intensity signal (specifically, a signal level of the light intensity signal) and the blood flow concentration N in the case where the light receiving element 12 receives the forward scattered light of the laser light LB. If the light receiving element 12 receives the forward scattered light of the laser light LB, the larger the blood flow concentration N is, the smaller the signal level of the light intensity signal is. In other words, if the light receiving element 12 receives the forward scattered light of the laser light LB, the smaller the blood flow concentration N is, the larger the signal level of the light intensity signal is. Namely, the more the blood includes the red blood cell which is the scattering substance to prevent the transmission of the laser light LB, the smaller the light intensity signal is.

If the light receiving element 12 receives the forward scattered light of the laser light LB, the processor 143 calculates the blood flow concentration N corresponding to the light intensity signal outputted from the A/D converter 142, by referring to a correlation information which is illustrated in FIG. 3(a) and which is stored in the inside or the outside of the processor 143 in advance. For example, if the light intensity signal outputted from the A/D converter 142 is P1, the processor 143 obtains the blood flow concentration N of "NH1" as a result of the calculation. On the other hand, if the light intensity signal outputted from the A/D converter 142 is P2 (P1 < P2), the processor 143 obtains the blood flow concentration N of "NL1 (NL1 < NH1)" as a result of the calculation.

On the other hand, FIG. 3(b) illustrates the correlation between the light intensity signal (specifically, a signal level of the light intensity signal) and the blood flow concentration N in the case where the light receiving element 12 receives the backward scattered light of the laser light LB. If the light receiving element 12 receives the backward scattered light of the laser light LB, the larger the blood flow concentration N is, the larger the signal level of the light intensity signal is. In other words, if the light receiving element 12 receives the backward scattered light of the laser light LB, the smaller the blood flow concentration N is, the smaller the signal level of the light intensity signal is. Namely, the more the blood includes the red blood cell which is the scattering substance to reflect the laser light LB, the larger the light intensity signal is.

If the light receiving element 12 receives the backward scattered light of the laser light LB, the processor 143 calculates the blood flow concentration N corresponding to the light intensity signal outputted from the A/D converter 142, by referring to a correlation information which is illustrated in FIG. 3(b) and which is stored in the inside or the outside of the processor 143 in advance. For example, if the light intensity signal outputted from the A/D converter 142 is P1, the processor 143 obtains the blood flow concentration N of "NL2" as a result of the calculation. On the other hand, if the light intensity signal outputted from the A/D converter 142 is P2 (P1 < P2), the processor 143 obtains the blood flow concentration N of "NH2 (NL2 < NH2)" as a result of the calculation.

Incidentally, FIG. 3(a) and FIG. 3(b) illustrate an example in which the correlation between the light intensity signal and the blood flow concentration N is represented by using the graph. However, the processor 143 may calculate the blood flow concentration N corresponding to the light intensity signal outputted from the A/D converter 142, by referring to an arbitrary information (for example, a mathematical formula, a table, a map or the like) which represents the correlation between the light intensity signal and the blood flow concentration N, in addition to or instead of the graph which represents the correlation between the light intensity signal and the blood flow concentration N.

Moreover, in the first example, the processor 143 calculates the blood flow concentration N on the basis of the correlation information which represents the correlation between the light intensity signal and the blood flow concentration N illustrated in FIG. 3(a) and FIG. 3(b). Therefore, the "blood flow concentration N" in the first example becomes synonym with a concentration of the blood cell (namely, the blood cell which is a scattering substance) included in the blood. However, another concentration (for example, a concentration of a substance other than the blood cell which is included in the blood) may be used as the blood flow concentration N.

Again in FIG. 2, subsequent to, in tandem with or in parallel with the operation of calculating the blood flow concentration N in the step S14 to the step S16, the HPF 144 cuts the signal component in the other frequency band other than the high frequency signal component from the signal component included in the output of the amplifier 13. As a result, the HPF 144 obtains the beat signal which corresponds to the high frequency signal component in the signal component included in the output of the amplifier 13 (step S17). Then, the HPF 144 outputs the obtained beat signal into the A/D converter 145.

Then, the A/D converter 145 performs the A/D converting process (namely, the quantizing process) on the beat signal outputted from the HPF 144 (step S18). Specifically, if a sampling cycle of the A/D converter 145 is Ta2, the A/D converter 145 outputs the sample value of the beat signal (namely, the quantized beat signal) every cycle Ta2. Then, the A/D converter 145 outputs the sample value of the beat signal (namely, the quantized beat signal) which is an interfering component of the scattered light which the light receiving element 12 receives into the processor 146.

Then, the processor 146 calculates the blood flow rate Q on the basis of the output of the A/D converter 145 (namely, the sample value of the beat signal which is the interfering component of the scattered light which the light receiving element 12 receives) and the output of the processor 143 (namely, the blood flow concentration N) (step S19).

(1-3) Operation of Calculating Blood Flow rate Q

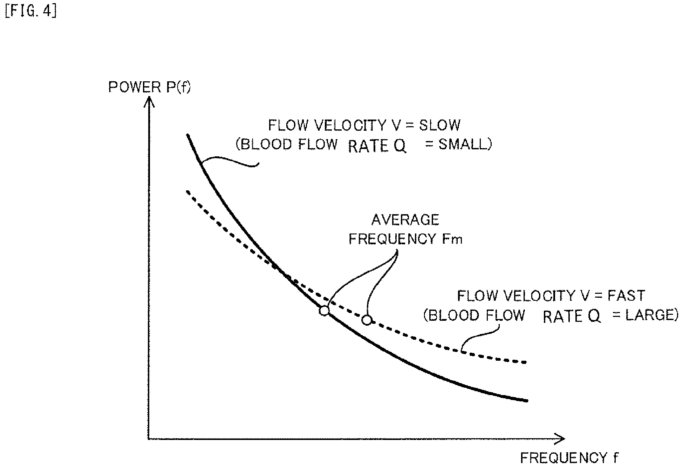

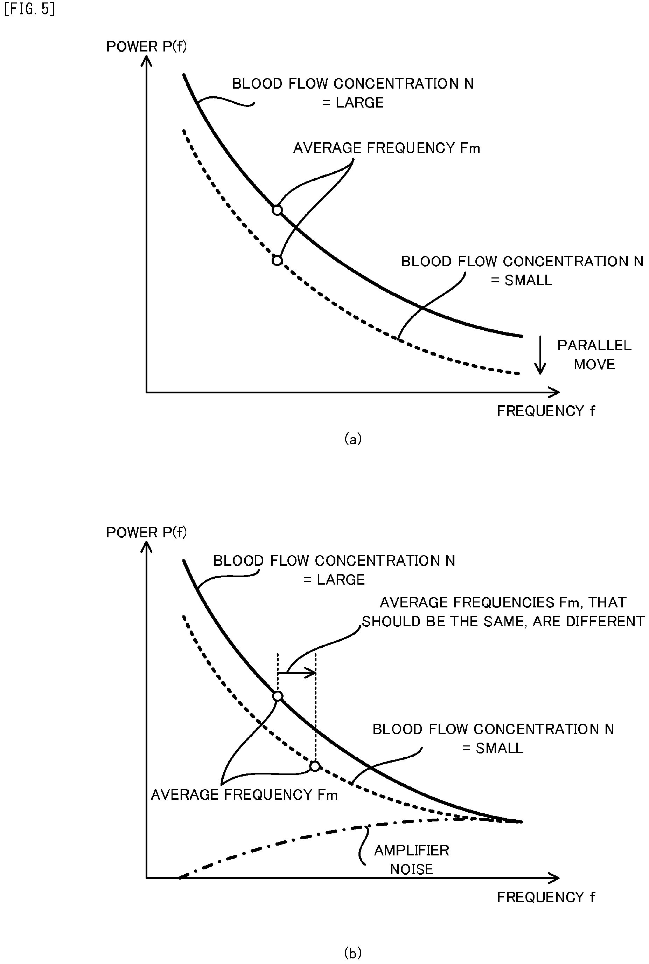

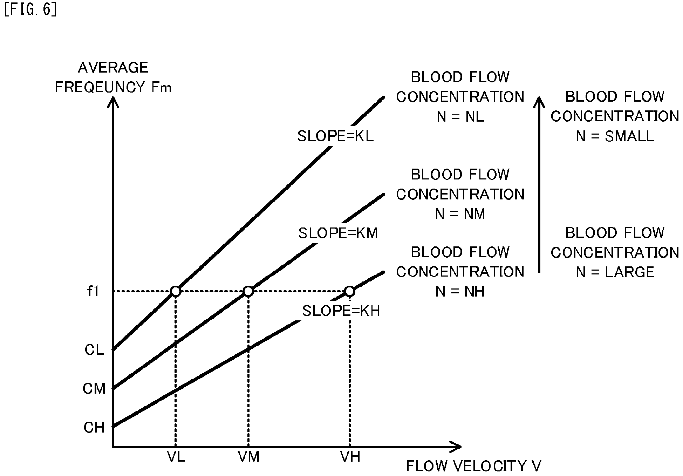

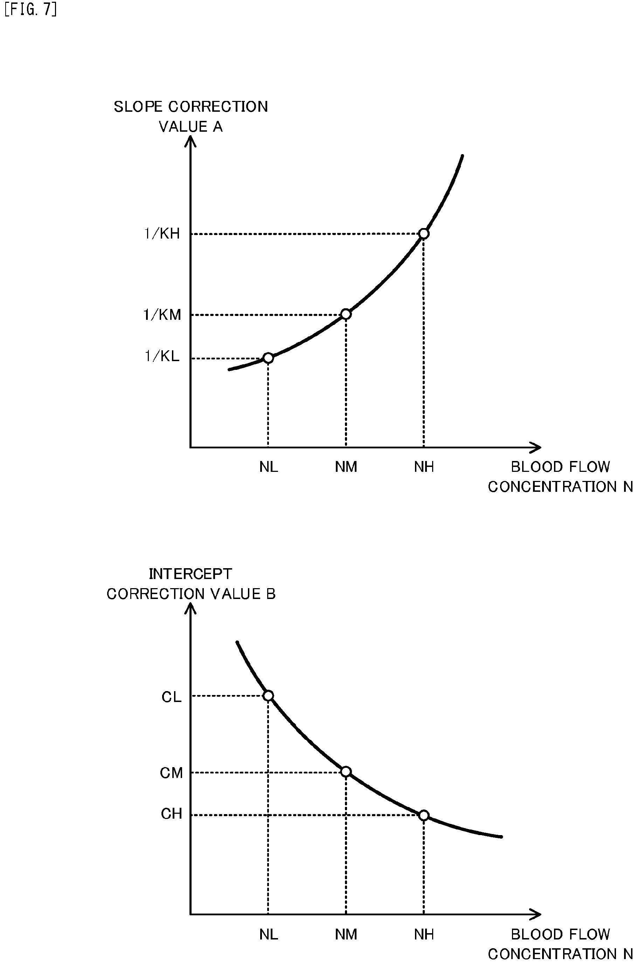

Next, with reference to FIG. 4 to FIG. 7, an operation of calculating the blood flow rate Q at the step S19 in FIG. 2. FIG. 4 is a graph illustrating a power spectrum (namely, a frequency f versus a power P(f) characteristics) which is obtained by performing the FFT on the sample value of the beat signal when the flow velocity v of the blood is relatively fast and a power spectrum which is obtained by performing the FFT on the sample value of the beat signal when the flow velocity v of the blood is relatively slow. FIGs. 5 are graphs illustrating both of an ideal value and an actual value of each of the power spectrum which is obtained by performing the FFT on the sample value of the beat signal when the blood flow concentration N is relatively large and the power spectrum which is obtained by performing the FFT on the sample value of the beat signal when the blood flow concentration N is relatively small. FIG. 6 is a graph illustrating a correlation between the flow velocity v of the blood and an average frequency Fm of the power spectrum for each blood flow concentration N. FIG. 7 is a graph illustrating a relationship between the blood flow concentration N and each of a slope correction value A and an intercept correction value B, wherein the slope correction value A and the intercept correction value B are used to calculate the flow velocity v of the blood from the average frequency Fm of the power spectrum.

In calculating the blood flow rate Q, the processor 146 firstly performs the FFT (the Fast Fourier Transform) on the output of the A/D converter 145 (namely, the sample value of the beat signal which is the interfering component of the scattered light which the light receiving element 12 receives). As a result, the processor obtains the power spectrum (namely, the power spectrum which represents the power P(f) of the beat signal for each frequency f) which is illustrated in FIG. 4.

As illustrated in FIG. 4, the power P(f) of the beat signal in a relatively low frequency range in the case where the flow velocity v of the blood is relatively fast (namely, the blood flow rate Q is relative large) is smaller than that in the case where the flow velocity v of the blood is relatively slow (namely, the blood flow rate Q is relative small). In addition, the power P(f) of the beat signal in a relatively high frequency range in the case where the flow velocity v of the blood is relatively fast (namely, the blood flow rate Q is relative large) is larger than that in the case where the flow velocity v of the blood is relatively slow (namely, the blood flow rate Q is relative small).

Considering the power spectrum which is illustrated in FIG. 4, the flow velocity v of the blood depends on the average frequency Fm on the power spectrum. Namely, the higher the average frequency Fm is, the faster the flow velocity v of the blood is. In other word, the lower the average frequency Fm is, the slower the flow velocity v of the blood is. Moreover, there is a positive correlation between the flow velocity v of the blood and the blood flow rate Q under such a condition that the tube 100 does not change. Namely, the faster the flow velocity v of the blood is, the larger the blood flow rate Q is. In other word, the slower the flow velocity v of the blood is, the smaller the blood flow rate Q is.

The processor 146 calculates the blood flow rate Q by considering the above described relationship among the average frequency Fm, the flow velocity v of the blood and the blood flow rate Q. Specifically, the processor 146 calculates the blood flow rate Q by using a formula 1. Incidentally, f means the frequency of the beat signal, and P(f) means the power of the beat signal whose frequency is f. Then, the processor 146 calculates the flow velocity v of the blood by using a formula 2. Incidentally, K means a coefficient for a flow velocity conversion. Then, the processor 146 calculates the blood flow rate Q by using a formula 3. Incidentally, L means a coefficient for a flow rate conversion.

[Formula 1]

$$Fm = \frac{\int f \times P(f)df}{\int P(f)df}$$

[Formula 2]

$$v = K \times Fm$$

[Formula 3]

$$Q = L \times v \times \int P(f)df$$

The processor 146 is capable of calculating the blood flow rate Q by using the formula 1 to the formula 3, if the processor 146 does not consider the blood flow concentration N at all. However, in the first [Formula 1] example, the processor 14b calculates the blood flow rate Q by considering the blood flow concentration N. This is why the power spectrum of the beat signal changes depending on the blood flow concentration N as illustrated in FIG. 5(a) and FIG. 5(b).

Specifically, FIG. 5(a) illustrates the ideal value (namely, a theoretical value) of each of the power spectrum (see a solid line) which is obtained by performing the FFT on the sample value of the beat signal when the blood flow concentration N is relatively large and the power spectrum (see a dashed line) which is obtained by performing the FFT on the sample value of the beat signal when the blood flow concentration N is relatively small. Namely, ideally (in other words, theoretically), the power spectrum moves along only a vertical axis (namely, an axis corresponding to the power) in parallel, without moving along a horizontal axis (namely, an axis corresponding to the frequency), if the blood flow concentration N changes. As a result, ideally, the average frequency Fm, which is calculated from the power spectrum, does not change depending on the blood flow concentration N. Therefore, ideally, the processor 146 should be capable of calculating the blood flow rate Q by using the formula 1 to the formula 3, whatever the blood flow concentration N is.

However, actually, as illustrated in FIG. 5(b), the power spectrum changes depending on the blood flow concentration N in a manner which is different from the manner illustrated in FIG. 5(a). Incidentally, FIG. 5(b) illustrates the actual value (namely, an actually measured value) of each of the power spectrum (see a solid line) which is obtained by performing the FFT on the sample value of the beat signal when the blood flow concentration N is relatively large and the power spectrum (see a dashed line) which is obtained by performing the FFT on the sample value of the beat signal when the blood flow concentration N is relatively small.

Actually, as illustrated in FIG. 5, the number of the blood cell, which is the scattering substance in the blood, per unit volume in the case where the blood flow concentration N is relatively large is more than that in the case where the blood flow concentration N is relatively small. Therefore, the power P(f) of the beat signal in the case where the blood flow concentration N is relatively large is larger than that in the case where the blood flow concentration N is relatively small, in all frequency range without exception. On the other hand, the number of the blood cell, which is the scattering substance in the blood, per unit volume in the case where the blood flow concentration N is relatively small is less than that in the case where the blood flow concentration N is relatively large. Therefore, the power P(f) of the beat signal in the case where the blood flow concentration N is relatively small is smaller than that in the case where the blood flow concentration N is relatively large, in all frequency range without exception.

However, an amplifier noise (specifically, an amplifier noise which is caused by a parasitic capacitance of an input terminal of the amplifier 13) which is illustrated by an alternate long and short dash line in FIG. 5(b) influences the power P(f) more in the case where the blood flow concentration N is relatively small, because the power P(f) of the beat signal relatively decreases when the blood flow concentration N is relatively small. The amplifier noise has such a characteristics that the amplifier noise becomes larger as the frequency f becomes higher. Thus, if the blood flow concentration N is relatively small, the amplifier noise is easily dominant to the power P(f) especially in the relatively high frequency range. Therefore, if the blood flow concentration N is relatively small, the power P(f) especially in the relatively high frequency range does not decrease as much as the ideal value (the theoretical value).

As a result, actually, the average frequency Fm, which is calculated from the power spectrum, changes depending on the blood flow concentration N. More specifically, the average frequency Fm in the case where the blood flow concentration N is relatively small is higher than the average frequency in the case where the blood flow concentration N is relatively large, even if the flow velocity v of the blood and the blood flow rate Q do not change. Therefore, it is preferable that the processor 146 calculate the blood flow rate Q with considering the blood flow concentration N, in the viewpoint of calculating the blood flow rate Q with relatively high accuracy. Thus, in the first example, the processor 146 calculates the blood flow rate Q with considering the blood flow concentration N.

Incidentally, a method of decreasing the influence of the amplifier noise by increasing the power of the laser light LB with which the laser element 11 irradiates (as a result, by increasing the power on the power spectrum) may be conceived. However, if the power of the laser light LB increases, such a disadvantage that a consumed power of the blood flow rate calculation apparatus 1 increases arises. Therefore, in the first example, the increase of the consumed power of the blood flow rate calculation apparatus 1 is prevented, because the blood flow rate Q is calculated by considering the blood flow concentration N.

Specifically, the processor 146 calculates the blood flow rate Q in which the blood flow concentration N is reflected, by using a correlation information which represents a correlation between the average frequency Fm and the flow velocity v of the blood for each blood flow concentration N. Incidentally, FIG. 6 represents a correlation between the average frequency Fm and the flow velocity v of the blood in the case where the blood flow concentration N is NL, a correlation between the average frequency Fm and the flow velocity v of the blood in the case where the blood flow concentration N is NM (NM > NL), and a correlation between the average frequency Fm and the flow velocity v of the blood in the case where the blood flow concentration N is NH (NH > NM > NL).

For example, if the blood flow concentration N which is outputted from the processor 143 is NL and the average frequency Fm which is calculated on the basis of the above described formula 1 is f1, the processor 146 obtains VL as the flow velocity v of the blood as a result of the calculation on the basis of the correlation information illustrated in FIG. 6. Then, the processor 146 calculates the blood flow rate Q by inputting the calculated flow velocity v = VL of the blood into the above described formula 3.

In a same manner, if the blood flow concentration N which is outputted from the processor 143 is NM and the average frequency Fm which is calculated on the basis of the above described formula 1 is f1, the processor 146 obtains VM (VM > VL) as the flow velocity v of the blood as a result of the calculation on the basis of the correlation information illustrated in FIG. 6. Then, the processor 146 calculates the blood flow rate Q by inputting the calculated flow velocity v = VM of the blood into the above described formula 3.

In a same manner, if the blood flow concentration N which is outputted from the processor 143 is NH and the average frequency Fm which is calculated on the basis of the above described formula 1 is f1, the processor 146 obtains VH (VH > VM) as the flow velocity v of the blood as a result of the calculation on the basis of the correlation information illustrated in FIG. 6. Then, the processor 146 calculates the blood flow rate Q by inputting the calculated flow velocity v = VH of the blood into the above described formula 3.

Incidentally, as illustrated in FIG. 6, the correlation information represents the correlation between the average frequency $Fm$ and the flow velocity $v$ of the blood such that the flow velocity $v$ of the blood becomes faster as the blood flow concentration $N$ becomes larger under such a condition that the average frequency $Fm$ does not change. Since the correlation information defines the correlation which has the above described characteristics, the influence of the amplifier noise is eliminated when the blood flow rate $Q$ is calculated.

Here, the correlation between the average frequency $Fm$ and the flow velocity $v$ of the blood is approximated by a liner expression (however, more precisely, the correlation between the average frequency $Fm$ and the flow velocity $v$ of the blood is represented by a formula other than the liner expression). Specifically, the correlation between the average frequency $Fm$ and the flow velocity $v$ of the blood in the case where the blood flow concentration $N$ is NL is approximated by such a formula that "flow velocity $v = (1 / \text{slope KL}) \times$ (average frequency $Fm$ - intercept CL)". In other words, the correlation between the average frequency $Fm$ and the flow velocity $v$ of the blood in the case where the blood flow concentration $N$ is NL is approximated by such a formula that "average frequency $Fm$ = slope KL $\times$ flow velocity $v$ + intercept CL". Moreover, the correlation between the average frequency $Fm$ and the flow velocity $v$ of the blood in the case where the blood flow concentration $N$ is NM is approximated by such a formula that "flow velocity $v = (1 / \text{slope KM (wherein KM < KL)}) \times$ (average frequency $Fm$ - intercept CM (wherein CM < CL))". In other words, the correlation between the average frequency $Fm$ and the flow velocity $v$ of the blood in the case where the blood flow concentration $N$ is NM is approximated by such a formula that "average frequency $Fm$ = slope KM $\times$ flow velocity $v$ + intercept CM". Moreover, the correlation between the average frequency $Fm$ and the flow velocity $v$ of the blood in the case where the blood flow concentration $N$ is NH is approximated by such a formula that "flow velocity $v = (1 / \text{slope KH (wherein KH < KM)}) \times$ (average frequency $Fm$ - intercept CH (wherein CH < CM))". In other words, the correlation between the average frequency $Fm$ and the flow velocity $v$ of the blood in the case where the blood flow concentration $N$ is NH is approximated by such a formula that "average frequency $Fm$ = slope KH $\times$ flow velocity $v$ + intercept CH".

Therefore, in the first example, the processor 146 may use a correlation information which represents a correlation between the blood flow concentration $N$ and each of the slope correction value A (an inverse number of the slope (namely, the slope KL, the slope KM and the slope KH) illustrated in FIG. 6, and see FIG. 7(a)) and the intercept correction value B (the intercept illustrated in FIG. 6, and see FIG. 7(b)), wherein the slope correction value A and the intercept correction value B are capable of defining the correlation information, in addition to or instead of directly using the correlation information illustrated in FIG. 6.

Specifically, a correlation information illustrated in FIG. 7(a) represents a correlation between the slope correction value A and the blood flow concentration $N$. Therefore, the processor 146 obtains $1 / KL$ as the slope correction value A as a result of the calculation by referring to the correlation information illustrated in FIG. 7(a), if the blood flow concentration $N$ which is outputted from the processor 143 is NL. In a same manner, the processor 146 obtains $1 / KM$ as the slope correction value A as a result of the calculation by referring to the correlation information illustrated in FIG. 7(a), if the blood flow concentration $N$ which is outputted from the processor 143 is NM. In a same manner, the processor 146 obtains $1 / KH$ as the slope correction value A as a result of the calculation by referring to the correlation information illustrated in FIG. 7(a), if the blood flow concentration $N$ which is outputted from the processor 143 is NH.

Moreover, a correlation information illustrated in FIG. 7(b) represents a correlation between the intercept correction value B and the blood flow concentration $N$. Therefore, the processor 146 obtains CL as the intercept correction value B as a result of the calculation by referring to the correlation information illustrated in FIG. 7(b), if the blood flow concentration $N$ which is outputted from the processor 143 is NL. In a same manner, the processor 146 obtains CM as the intercept correction value B as a result of the calculation by referring to the correlation information illustrated in FIG. 7(b), if the blood flow concentration $N$ which is outputted from the processor 143 is NM. In a same manner, the processor 146 obtains CH as the intercept correction value B as a result of the calculation by referring to the correlation information illustrated in FIG. 7(b), if the blood flow concentration $N$ which is outputted from the processor 143 is NH.

When the processor 146 uses the correlation information illustrated in FIG. 7(a) and FIG. 7(b), the processor 146 calculated the average frequency $Fm$ by using the above described formula 1. Then, the processor 146 calculates the flow velocity $v$ of the blood by inputting the calculated average frequency $Fm$, the slope correction value A which is calculated from the correlation information illustrated in FIG. 7(a) and the intercept correction value B which is calculated from the correlation information illustrated in FIG. 7(b) into a formula 4. Then, the processor 146 calculates the blood flow rate $Q$ by inputting the calculated flow velocity $v$ of the blood into the above described formula 3.

[Formula 4]

$$v = A \times (Fm - B)$$

Incidentally, it is preferable that the blood flow rate calculation apparatus 1 store therein the correlation information illustrated in FIG. 6 or the correlation information illustrated in FIG. 7(a) and FIG. 7(b) in advance. For example, the blood flow rate calculation apparatus 1 may generate the above described correlation information by calculating the blood flow rate Q and the like of a plurality of types of blood whose blood flow concentration are different from each other in advance.

Moreover, FIG. 6, FIG. 7(a) and FIG. 7(b) illustrate an example in which the correlation is represented by the graph. However, the processor 146 may calculates the blood flow rate Q by referring to an arbitrary information (for example, a formula, a table, a map or the like) which represents the correlation information, in addition to or instead of the graph which represents the correlation information.

As described above, the blood flow rate calculation apparatus 1 in the first example is capable of calculating the blood flow rate Q appropriately (in other words, with high accuracy) while considering the blood flow concentration N.

Incidentally, the above described operation of calculating the blood flow rate Q with considering the blood flow concentration N (namely, the operation of calculating the blood flow rate Q by using the formula 1, formula 3 and formula 4, and FIG. 6 and FIG. 7) is one example. Therefore, the blood flow rate calculation apparatus 1 may calculate the blood flow rate Q with considering the blood flow concentration N in a different manner. The blood flow rate calculation apparatus 1 may calculate the blood flow rate Q in an arbitrary manner, as long as the blood flow rate calculation apparatus 1 is capable of calculating the blood flow rate Q such that the influence of the amplifier noise due to the variation of the blood flow concentration N is eliminated.

Moreover, as described above, the frequency of the light intensity signal which is used to calculate the blood flow concentration N is typically equal to or less than 1 kHz and the frequency of the beat signal which is used to calculate the blood flow rate Q is typically equal to or more than 1 kHz. Therefore, the sampling cycle (namely, the cycle with which the quantization is performed) of the A/D converter 142 which is used to calculate the blood flow concentration N may be longer than the sampling cycle of the A/D converter 145 which is used to calculate the blood flow rate Q. In a same manner, a processing cycle (namely, a cycle with which blood flow concentration N is calculated) of the processor 143 which is used to calculate the blood flow concentration N may be longer than a processing cycle (namely, a cycle with which blood flow rate Q is calculated) of the processor 146 which is used to calculate the blood flow rate Q.

Moreover, in the above described explanation, the blood flow rate calculation apparatus 1 calculates both of the blood flow rate Q and the flow velocity v. However, there is a position correlation between the blood flow rate Q and the flow velocity v under such a condition that the tube 100 does not change. Namely, the larger the blood flow rate Q of the blood which flows in a certain tube 100 is, the faster the flow velocity v of the blood which flows in the certain tube 100 is. On the other hand, the smaller the blood flow rate Q of the blood which flows in a certain tube 100 is, the slower the flow velocity v of the blood which flows in the certain tube 100 is. Therefore, the calculation of the blood flow rate Q becomes synonym with the calculation of the flow velocity v. Namely, the calculation of the flow velocity v substantially corresponds to the indirect calculation of the blood flow rate Q, and the calculation of the blood flow rate Q substantially corresponds to the indirect calculation of the flow velocity v. Therefore, the blood flow rate calculation apparatus 1 may calculate one of the blood flow rate Q and the flow velocity v and may not calculate the other of the blood flow rate Q and the flow velocity v.

(2) Second Example

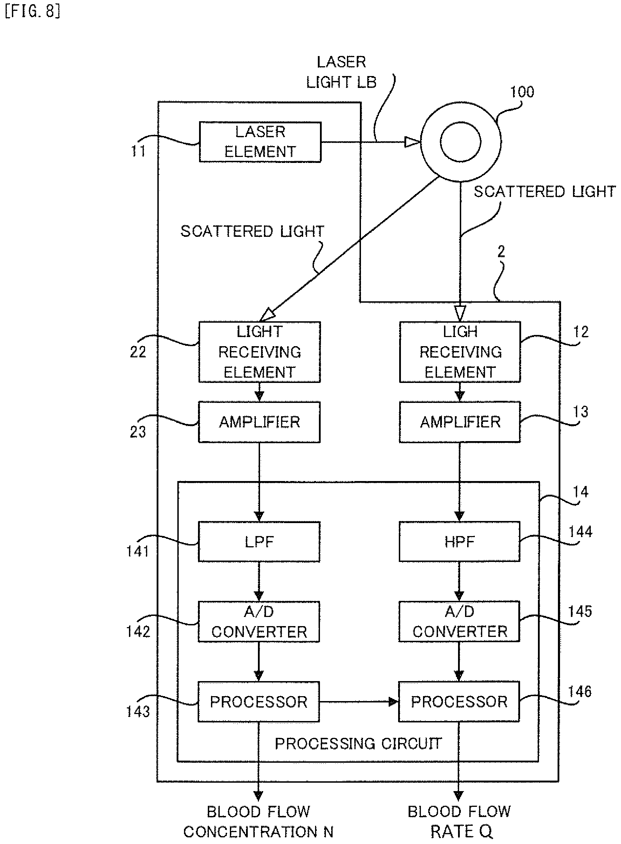

Next, with reference to FIG. 8, a blood flow rate calculation apparatus 2 in a second example will be explained. FIG. 8 is a block diagram illustrating the configuration of the blood flow rate calculation apparatus 2 in the second example. Incidentally, in the following example, regarding a feature and an operation which are same as the feature and the operation of the blood flow rate calculation apparatus 1 in the first example, same reference number and same step number are added and their explanation will be omitted.

As illustrated in FIG. 8, the blood flow rate calculation apparatus 2 in the second example is different from the blood flow rate calculation apparatus 1 in the first example in that the blood flow rate calculation apparatus 2 is further provided with: a light receiving element 22 and an amplifier 23. Furthermore, the blood flow rate calculation apparatus 2 in the second example is different from the blood flow rate calculation apparatus 1 in the first example in that the LPF 141 cuts the signal component in the other frequency band other than the low frequency signal component from the signal component included in the output of the amplifier 23 (namely, in the received light signal corresponding to the scattered light which the light receiving element 22 receives) and the HPF 144 cuts the signal component in the other frequency band other than the high frequency signal component from the signal component included in the output of the amplifier 13 (namely, in the received light signal corresponding to the scattered light which the light receiving element 12 receives). Another feature which the blood flow rate calculation apparatus 2 in the second example is provided with may be same as another feature which the blood flow rate calculation apparatus 1 in the first example is provided with.

The light receiving element 22 receives the scattered light of the laser light LB with which the tube 100 is irradiated. The light receiving element 22 generates the received light signal which is obtained by converting the received scattered light into an electric signal.

Incidentally, the light receiving element 22 may receive the forward scattered light which corresponds to the transmitted light of the laser light LB with which the tube 100 is irradiated, and the light receiving element 12 may receive the backward scattered light which corresponds to the reflected light of the laser light LB with which the tube 100 is irradiated. Alternatively, the light receiving element 22 may receive the backward scattered light which corresponds to the reflected light of the laser light LB with which the tube 100 is irradiated, and the light receiving element 12 may receive the forward scattered light which corresponds to the transmitted light of the laser light LB with which the tube 100 is irradiated. Both of the light receiving element 12 and the light receiving element 22 may receive the forward scattered light which corresponds to the transmitted light of the laser light LB with which the tube 100 is irradiated. Both of the light receiving element 12 and the light receiving element 22 may receive the backward scattered light which corresponds to the reflected light of the laser light LB with which the tube 100 is irradiated.

The amplifier 23 converts the received light signal which is an electric current signal outputted from the light receiving element 22 into a voltage signal and amplifies the voltage signal.

The blood flow rate apparatus 2 in the second example is capable of enjoying the effect which is same as the effect which the blood flow rate apparatus 1 in the first example is capable of enjoying.

In addition, according to the blood flow rate apparatus 2 in the second example, the light receiving element 12 which is mainly used to calculate the blood flow rate Q and the light receiving element 22 which is mainly used to calculate the blood flow concentration N are prepared separately and independently.

Thus, it is possible to make a light receiving area of the light receiving element 12, which is mainly used to calculate the blood flow rate Q, to be relatively small. The light receiving area of the light receiving element 12, which is mainly used to calculate the blood flow rate Q, is made smaller than the light receiving area of the light receiving element 22, which is mainly used to calculate the blood flow concentration N. As a result, it is possible to relatively reduce the influence of the amplifier noise which is a factor to deteriorate the accuracy of calculating the blood flow rate Q (for example, to relatively reduce the parasitic capacitance of the input terminal of the amplifier 13 which is a factor to generate the noise).

On the other hand, it is possible to make the light receiving area of the light receiving element 22, which is mainly used to calculate the blood flow concentration N, to be relatively large. The light receiving area of the light receiving element 22, which is mainly used to calculate the blood flow concentration N, is made larger than the light receiving area of the light receiving element 12, which is mainly used to calculate the blood flow rate Q. As a result, it is possible to relatively improve a S/N ratio of the light intensity signal which is used to calculate the blood flow concentration N.

Incidentally, a distance between the laser element 11 and the light receiving element 12 whose light receiving area can be relatively small may be shorter than a distance between the laser element 11 and the light receiving element 22 whose light receiving area can be relatively large such that both of the light receiving element 12 and the light receiving element 22 are capable of receiving the scattered light of the laser light LB appropriately. In other words, it is preferable that the light receiving element 12 whose light receiving area can be relatively small be as close as possible to the laser element 11.

(3) Third Example

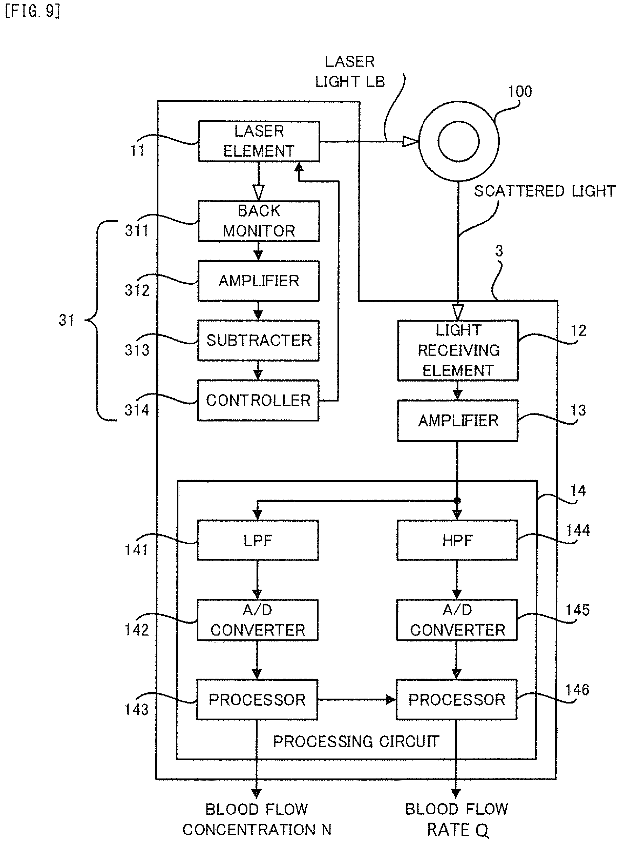

Next, with reference to FIG. 9, a blood flow rate calculation apparatus 3 in a third example will be explained. FIG. 9 is a block diagram illustrating the configuration of the blood flow rate calculation apparatus 3 in the third example. Incidentally, in the following example, regarding a feature and an operation which are same as the feature and the operation of the blood flow rate calculation apparatus 1 in the first example, same reference number and same step number are added and their explanation will be omitted.

As illustrated in FIG. 9, the blood flow rate calculation apparatus 3 in the third example is different from the blood flow rate calculation apparatus 1 in the first example in that the blood flow rate calculation apparatus 3 is further provided with an APC (Automatic Power Control) circuit 31. Another feature which the blood flow rate calculation apparatus 3 in the third example is provided with may be same as another feature which the blood flow rate calculation apparatus 1 in the first example is provided with.

The APC circuit 31 is provided with: a back monitor 311; an amplifier 312; a subtracter (a subtracting circuit) 313; and a controller 314.

The back monitor 311 receives the laser light LB which is emitted from the laser element 11 (especially, the laser light LB leaked from an edge surface which is opposite to an emitting edge surface of the laser element 11). The back monitor 311 generate a received light signal which is obtained by converting the received laser light LB into an electric signal.

The amplifier 312 converts the received light signal which is an electric current signal outputted from the back monitor 311 into a voltage signal and amplifies the voltage signal.

The subtracter 313 subtracts the voltage signal which is outputted from the amplifier 312 from a predetermined target signal (specifically, a signal which corresponds to a target power of the laser light LB which is emitted from the laser element 11). Namely, the subtracter 313 calculates a deviation (difference, error) between a power of the laser light LB which is emitted from the laser element 11 and the target power. The subtracter 313 outputs a deviation signal which represents the deviation into the controller 314.

The controller 314 performs a phase compensation, which is to perform a stable negative feedback, on the deviation signal which is outputted from the subtracter 313. Then, the controller 314 controls the laser element 11 on the basis of the deviation signal on which the phase compensation is performed. As a result, the laser element 11 emits the laser light LB whose power is changed by an amount corresponding to the deviation signal on which the phase compensation is performed.

The blood flow rate apparatus 3 in the third example is capable of enjoying the effect which is same as the effect which the blood flow rate apparatus 1 in the first example is capable of enjoying.

In addition, according to the blood flow rate apparatus 3 in the third example, the APC circuit 31 is capable of making the power of the laser light LB which is emitted from the laser element 11 to be same as the target power. Therefore, the laser light LB is capable of emitting the laser light LB stably. As a result, the blood flow rate calculation apparatus 3 is capable of calculating the blood flow rate Q and the blood flow concentration N more appropriately (in other words, with higher accuracy) by using the stable laser light LB.

Incidentally, the blood flow rate calculation apparatus 3 may be provided with a front monitor which receives the laser light LB which is emitted from the laser element 11 through a beam splitter, in addition to or instead of the back monitor 311.

(4) Fourth Example

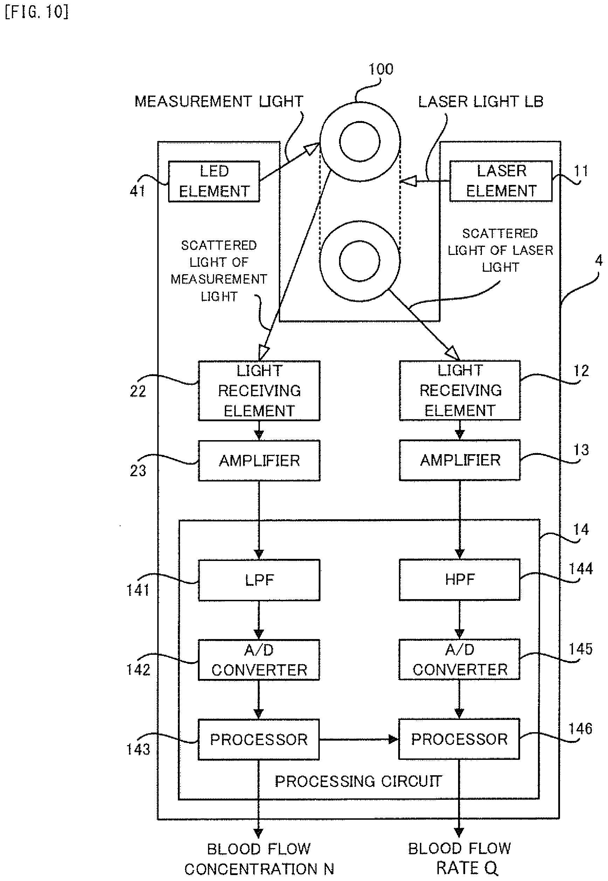

Next, with reference to FIG. 10, a blood flow rate calculation apparatus 4 in a fourth example will be explained. FIG. 10 is a block diagram illustrating the configuration of the blood flow rate calculation apparatus 4 in the fourth example. Incidentally, in the following example, regarding a feature and an operation which are same as the feature and the operation of the blood flow rate calculation apparatus 2 in the second example, same reference number and same step number are added and their explanation will be omitted.

As illustrated in FIG. 10, the blood flow rate calculation apparatus 4 in the fourth example is different from the blood flow rate calculation apparatus 2 in the second example in that the blood flow rate calculation apparatus 4 is further provided with a LED element 41. Another feature which the blood flow rate calculation apparatus 4 in the fourth example is provided with may be same as another feature which the blood flow rate calculation apparatus 2 in the second example is provided with.

The LED element 41 irradiates the tube 100 with a measurement light which is mainly used to calculate the blood flow concentration N. It is preferable that the LED element 41 irradiate the flow passage (namely, the flow passage in which the blood flows) in the tube 100 with the measurement light. Moreover, it is preferable that the wavelength of the measurement light be 805 nanometer which is not affected by a degree of oxygen saturation in the blood, in order to decreasing the influence of the degree of the oxygen saturation in the blood in calculating the blood flow concentration N.

On the other hand, the laser element 11 irradiates the tube 100 with the laser light LB which is mainly used to calculate the blood flow rate Q. Here, when the blood flow rate Q is calculated, the beat signal which is caused by the mutual interference between the scattered lights. Therefore, it is preferable that the light with which the tube 100 is irradiated to calculate the blood flow rate Q have a high monochromatic characteristics and a high coherency. Therefore, the laser element 11 emits the laser light LB having the above described characteristics in order to calculate the blood flow rate Q. Incidentally, the wavelength of the laser light LB may be 780 nanometer in the viewpoint of easy preparation of the laser element 11.

Incidentally, it is preferable that an area on the tube 100 which the laser element 11 irradiates with the laser light LB be different from an area on the tube 100 which the LED element 41 irradiates with the measurement light, to prevent the interference and so on between the laser light LB and the measurement light. In other words, it is preferable that the area on the tube 100 which the laser element 11 irradiates with the laser light LB be distant by a predetermined distance from the area on the tube 100 which the LED element 41 irradiates with the measurement light, to prevent the interference and so on between the laser light LB and the measurement light. Alternatively, the mutual interference can be prevented by adjusting an irradiating timing of each of the laser light LB and the measurement light.

In the fourth example, the light receiving element 12 receives the scattered light of the laser light LB with this the laser element 11 irradiates the tube 100. On the other hand, the light receiving element 22 receives the scattered light of the measurement light with this the LED element 41 irradiates the tube 100.

The blood flow rate apparatus 4 in the fourth example is capable of enjoying the effect which is same as the effect which the blood flow rate apparatus 2 in the second example is capable of enjoying.

In addition, according to the blood flow rate apparatus 4 in the fourth example, the laser element 11 which is mainly used to calculate the blood flow rate Q and the LED element 41 which is mainly used to calculate the blood flow concentration N are prepared separately and independently. Thus, the blood flow rate Q is calculated by using the laser light LB which is suitable for calculating the blood flow rate Q and the blood flow concentration N is calculated by using the measurement light which is suitable for calculating the blood flow concentration N. Therefore, the blood flow rate apparatus 4 is capable of calculating the blood flow rate Q and the blood flow concentration N more appropriately.

(5) Specific Example of Light Receiving Element 12 and Amplifier 13

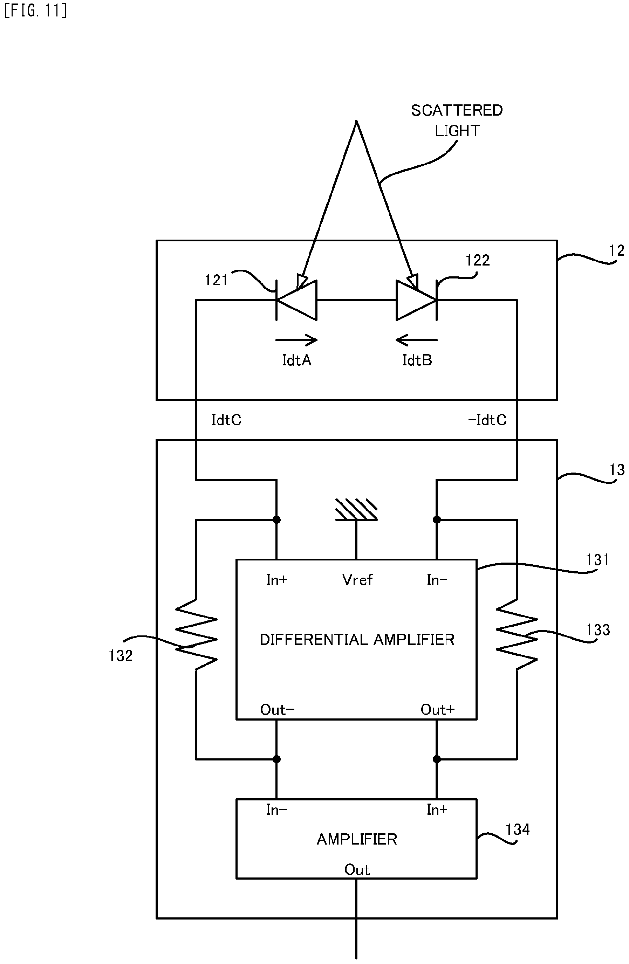

Next, with reference to FIG. 11, a specific example of the light receiving element 12 and the amplifier 13 which each of the blood flow rate calculation apparatus 1 in the first example to the blood flow rate calculation apparatus 4 in the fourth example is provided with will be explained. FIG. 11 is a block diagram illustrating a configuration of a specific example of the light receiving element 12 and the amplifier 13 which each of the blood flow rate calculation apparatus 1 in the first example to the blood flow rate calculation apparatus 4 in the fourth example is provided with.

As illustrated in FIG. 11, the light receiving element 12 is provided with: a PIN type photo diode 121; and a PIN type photo diode 122. The PIN type photo diode 121 and the PIN type photo diode 122 are coupled in series such that anodes of the PIN type photo diode 121 and the PIN type photo diode 122 are coupled with each other. A cathode of the PIN type photo diode 121 is coupled to the amplifier 13 (especially, a normal phase input terminal of a differential amplifier 131 which the amplifier 13 is provided with). A cathode of the PIN type photo diode 122 is coupled to the amplifier 13 (especially, a reverse phase input terminal of the differential amplifier 131 which the amplifier 13 is provided with).

The light receiving element 12 outputs, as the received light signal, a differential electric current IdtC (=IdtB - IdtA) between an electric current IdtA outputted from the PIN type photo diode 121 and an electric current IdtB outputted from the PIN type photo diode 122 into the amplifier 13 (especially, the normal phase input terminal of the differential amplifier 131 which the amplifier 13 is provided with). Moreover, the light receiving element 12 outputs, as the received light signal, a differential electric current -IdtC which is obtained by inverting a polarity of the differential electric current IdtC into the amplifier 13 (especially, the reverse phase input terminal of the differential amplifier 131 which the amplifier 13 is provided with).

According to the light receiving element 12 having the above described configuration, a DC component of the electric current IdtA outputted from the PIN type photo diode 121 is canceled by a DC component of the electric current IdtB outputted from the PIN type photo diode 122. Therefore, the light receiving element 12 is capable of outputting, as the received light signal, the differential electric current IdtC mainly including an AC component which corresponds to a signal component included in the scattered light (namely, the beat signal). Thus, the S/N ratio in the amplifier 13 can be improved relatively.

The amplifier 13 is provided with: the differential amplifier 131; a feedback resistor 132; a feedback resister 133; and a next step amplifier 134.

The differential amplifier 131 converts the differential electric current IdtC, which is inputted to the normal input terminal In+, into the voltage signal and outputs the voltage signal from a reverse output terminal Out-. In a same manner, the differential amplifier 131 converts the differential electric current -IdtC, which is inputted to the reverse input terminal In-, into the voltage signal and outputs the voltage signal from a normal output terminal Out+. Namely, the differential amplifier 131 functions as a transimpedance amplifier which converts the electric current inputted to the normal input terminal In+ and reverse input terminal In- into the voltages separately and differentially output the voltages. Incidentally, a reference voltage of the differential amplifier 131 is inputted to the differential amplifier 131 via a reference voltage terminal Vref.

Here, the feedback resister 132 which is located between the normal input terminal In+ and the reverse output terminal Out-performs a negative feedback. Moreover, the feedback resister 133 which is located between the reverse input terminal In- and the normal output terminal Out+ performs a negative feedback. Therefore, a difference of voltage between the normal input terminal In+ and the reference voltage terminal Vref is zero (or a value which can be treated as zero). In a same manner, a difference of voltage between the reverse input terminal In- and the reference voltage terminal Vref is zero (or a value which can be treated as zero). Therefore, the voltage of the normal input terminal In+ and the voltage of the reverse input terminal In- are the same (or in such a condition that both can be treated to be the same). Therefore, a voltage of the cathode of the PIN type photo diode 121 which is coupled to the normal input terminal In+ and a voltage of the cathode of the PIN type photo diode 122 which is coupled to the reverse input terminal In- are the same (or in such a condition that both can be treated to be the same). Thus, each of the PIN type photo diode 121 and the PIN type photo diode 122 operates in a zero bias condition. Therefore, a dark electric current which is generated in the laser element 11 can be reduced or eliminated. Thus, the S/N ratio in the amplifier 13 can be improved relatively, because the noise caused by a fluctuation of the dark electric current is reduced.

Incidentally, the next step amplifier 134 functions as what we call an instrumentation amplifier and amplifies and outputs the differential signal which is outputted from the differential amplifier 131.

Incidentally, one part of the features which are explained in the first example to the fourth example can be combined as occasion demand. Even in this case, the blood flow rate calculation apparatus, which is obtained by combining one part of the features which are explained in the first example to the fourth example, is capable of enjoying the above described various effects.

Description of Reference Signs

1, 2, 3, 4    blood flow rate calculation apparatus

| 11 | laser element |
| 12, 22 | light receiving element |
| 13, 23 | amplifier |
| 141 | LPF |
| 144 | HPF |
| 142, 145 | A/D converter |
| 143, 146 | processor |
| 311 | back monitor |
| 312 | amplifier |
| 313 | subtracter |
| 314 | controller |
| 41 | LED element |

## Claims

1. A fluid assessment apparatus (1) comprising:

    an irradiating device (11) which is configured to irradiate measurement target (100) with at least a laser light (LB), wherein a fluid flows in an interior of the measurement target; and
    a light receiving device (12) which is configured to receive at least the laser light with which the measurement target is irradiated;
    a first calculating device (143) which is configured to calculate a concentration (N) of the fluid from a first received light signal based on a signal intensity of a received light signal which the light receiving device receives; and
    a second calculating device (146) which is configured to calculate a flow rate (Q) of the fluid and / or a flow velocity (v) of the fluid from (i) a second received light signal based on a variation of a frequency of the received light signal which is caused by a Doppler shift of the laser light with which the measurement target is irradiated and (ii) the concentration which the first calculating device calculates,
    the light receiving device including a first photo diode (121) and a second photo diode (122) each of which is configured to receive the laser light and convert the laser light to electrical current (IdtA, IdtB),
    **characterized in that**:

    the first and second photo diodes being coupled in series such that same polarity nodes of the first and second photo diodes are coupled with each other,
    the light receiving device being configured to output, as the second received light signal, differential electrical current (IdtC) between the electrical current (IdtA) outputted from the first photo diode and the electrical current (IdtB) outputted from the second photo diode,
    the second calculating device including an amplifier (13) that is configured to amplify and convert the second light received light signal into voltage signal and output the voltage signal,
    the signal intensity is a signal intensity of a signal component of the received light signal whose frequency is relatively low; and
    the variation of the frequency is a variation of a frequency of a signal component of the received light signal whose frequency is relatively high,
    the light receiving device includes: (i) a first light receiving device (22) which obtains the first received light signal by receiving the laser light with which the measurement target is irradiated and (ii) a second light receiving device (12) which obtains the second received light signal by receiving the laser light with which the measurement target is irradiated,
    a light receiving area of the first light receiving device (22) is larger than a light receiving area of the second light receiving device (12).

2. The fluid assessment apparatus (1) according to claim1, wherein

    the irradiating device includes: (i) a first irradiating device (41) which irradiates the measurement target with a measurement light which is a light whose type is different from the type of the laser light; and (ii) a second irradiating device (11) which irradiates the measurement target with the laser light (LB),
    the light receiving device (12) (i) obtains the first received light signal by receiving the measurement light with which the measurement target is irradiated and (ii) obtains the second received light signal by receiving the laser light with which the measurement target is irradiated,
    the first calculating device (143) is configured to calculate the concentration (N) from the first received light signal,
    the second calculating device (146) is configured to calculate the flow rate (Q) and / or the flow velocity (v) from (i) the second received light signal and (ii) the concentration which the first calculating device calculates.

3. The fluid assessment apparatus (1) according to claim 1, wherein

    the first calculating device (143) is configured to calculate the concentration (N) from the first received light signal,
    the second calculating device (146) is configured to calculate the flow rate (Q) and / or the flow velocity (v) from (i) the second received light signal and (ii) the concentration which the first calculating device calculates.

4. The fluid assessment apparatus (1) according to claim 2, wherein

   the light receiving device includes: (i) a first light receiving device (22) which obtains the first received light signal by receiving the measurement light with which the measurement target is irradiated and (ii) a second light receiving device (12) which obtains the second received light signal by receiving the laser light with which the measurement target is irradiated,
   the first calculating device (143) is configured to calculate the concentration (N) from the first received light signal,
   the second calculating device (146) is configured to calculate the flow rate (Q) and / or the flow velocity (v) from (i) the second received light signal and (ii) the concentration which the first calculating device calculates.

5. The fluid assessment apparatus (1) according to claim 1, wherein
   the second calculating device (143) is configured to calculate the flow rate (Q) and / or the flow velocity (v) on the basis of correlation information, wherein the correlation information defines at least a first correlation between the variation of the frequency and the flow rate and / or the flow velocity in the case where the concentration (N) is first value and a second correlation between the variation of the frequency and the flow rate and / or the flow velocity in the case where the concentration is second value.

6. The fluid assessment apparatus (1) according to claim 1, wherein
   the second calculating device (146) is configured to calculate the flow rate (Q) and / or the flow velocity (v) on the basis of correlation information, wherein the correlation information defines at least a first correlation between an average frequency (Fm) which can be obtained by analyzing the second received light signal and the flow rate and / or the flow velocity in the case where the concentration (N) is first value and a second correlation between the average frequency and the flow rate and / or the flow velocity in the case where the concentration is second value.

7. The fluid assessment apparatus (1) according to claim 6, wherein
   the correlation information defines the first and second correlations representing that the flow rate (Q) and / or the flow velocity (v) which is calculated when the concentration (N) is the first value is smaller than the flow rate and / or the flow velocity which is calculated when the concentration is the second value that is larger than the first value under such a condition that the average frequency (Fm) does not change.

8. The fluid assessment apparatus (1) according to claim 1, wherein

   the second calculating device (146) is configured to calculate the flow rate (Q) and / or the flow velocity (v) with second cycle,
   the first calculating device (143) is configured to calculate the concentration (N) with first cycle that is longer than the second cycle.

9. The fluid assessment apparatus (1) according to claim 1, wherein
   the fluid is a blood of a living body, wherein one portion of the living body is the measurement target.

10. The fluid assessment apparatus (1) according to claim 1, wherein

    the first received light signal is generated from the received light signal of the laser light passing through the measurement target (100),
    the second received light signal is generated the received light signal of the laser light reflected by the measurement target (100).

11. A fluid assessment method using a fluid assessment apparatus (1), the fluid assessment apparatus comprising:

    an irradiating device (11) which is configured to irradiate measurement target (100) with at least a laser light (LB), wherein a fluid flows in an interior of the measurement target; and
    a light receiving device (12) which is configured to receive at least the laser light with which the measurement target is irradiated,
    a first calculating process which calculates a concentration (N) of the fluid from a first received light signal based on a signal intensity of a received light signal which the light receiving device receives; and
    a second calculating process which calculates a flow rate (Q) of the fluid and / or a flow velocity (v) of the fluid from (i) a second received light signal based on a variation of a frequency of the received light signal which is caused by a Doppler shift of the laser light with which the measurement target is irradiated and (ii) the concentration which the first calculating process calculates,
    the light receiving device including a first photo diode (121) and a second photo diode (122) each of which is configured to receive the laser light and convert the laser light to electrical current (IdtA, IdtB),

**characterized in that**:

the first and second photo diodes being coupled in series such that same polarity nodes of the first and second photo diodes are coupled with each other,

the light receiving device being configured to output, as the second received light signal, differential electrical current (IdtC) between the electrical current (IdtA) outputted from the first photo diode and the electrical current (IdtB) outputted from the second photo diode,

the second calculating process amplifying and converting the second light received light signal into voltage signal and output the voltage signal,

the signal intensity is a signal intensity of a signal component of the received light signal whose frequency is relatively low; and

the variation of the frequency is a variation of a frequency of a signal component of the received light signal whose frequency is relatively high,

the light receiving device includes: (i) a first light receiving device (22) which obtains the first received light signal by receiving the laser light with which the measurement target is irradiated and (ii) a second light receiving device (12) which obtains the second received light signal by receiving the laser light with which the measurement target is irradiated,

a light receiving area of the first light receiving device (22) is larger than a light receiving area of the second light receiving device (12).

**Patentansprüche**

1. Eine Fluid-Erfassungs-Vorrichtung (1), aufweisend:

eine Bestrahlungs-Vorrichtung (11), welche eingerichtet ist, ein Mess-Zielobjekt (100) zumindest mit einem Laser-Licht (LB) zu bestrahlen, wobei ein Fluid in einem Inneren des Mess-Zielobjekts fließt; und

eine Licht-Empfangs-Vorrichtung (12), welche eingerichtet ist, zumindest das Laser-Licht zu empfangen, mit welchem das Mess-Zielobjekt bestrahlt wird;

eine erste Ermittlungs-Vorrichtung (143), welche eingerichtet ist, eine Konzentration (N) des Fluids zu ermitteln anhand eines ersten empfangenen Lichtsignals basierend auf einer Signalintensität eines empfangenen Lichtsignals, welches die Licht-Empfangs-Vorrichtung empfängt; und

eine zweite Ermittlungs-Vorrichtung (146), welche eingerichtet ist, eine Flussrate (Q) des Fluids und/oder eine Strömungsgeschwindigkeit (v) des Fluids zu ermitteln anhand (i) eines zweiten empfangenen Lichtsignals basierend auf einer Variation einer Frequenz des empfangenen Lichtsignals, welches durch eine Dopplerverschiebung des Laser-Lichts, mit welchem das Mess-Zielobjekt bestrahlt wird, verursacht wird, und (ii) der Konzentration, welche die erste Ermittlungs-Vorrichtung ermittelt,

wobei die Licht-Empfangs-Vorrichtung eine erste Fotodiode (121) und eine zweite Fotodiode (122) aufweist, von welchen jede eingerichtet ist, das Laser-Licht zu empfangen und das Laser-Licht in elektrischen Strom (IdtA, IdtB) umzuwandeln,

**dadurch gekennzeichnet, dass**:

die erste Fotodiode und die zweite Fotodiode derart in Reihe geschaltet sind, dass Knoten gleicher Polarität der ersten Fotodiode und der zweiten Fotodiode miteinander gekoppelt sind,

die Licht-Empfangs-Vorrichtung eingerichtet ist, als das zweite empfangene Lichtsignal einen elektrischen Differenzstrom (IdtC) zwischen dem von der ersten Fotodiode ausgegebenen elektrischen Strom (IdtA) und dem von der zweiten Fotodiode ausgegebenen elektrischen Strom (IdtB) auszugeben,

die zweite Ermittlungs-Vorrichtung einen Verstärker (13) aufweist, der eingerichtet ist, das zweite empfangene Lichtsignal in ein Spannungssignal umzuwandeln, zu verstärken und das Spannungssignal auszugeben,

die Signalintensität eine Signalintensität einer Signalkomponente des empfangenen Lichtsignals ist, de ssen Frequenz relativ niedrig ist; und

die Variation der Frequenz eine Variation einer Frequenz einer Signalkomponente des empfangenen Lichts ignals ist, dessen Frequenz relativ hoch ist,

die Licht-Empfangs-Vorrichtung aufweist: (i) eine erste Licht-Empfangs-Vorrichtung (22), welche das erste empfangene Lichtsignal erlangt durch Empfangen des Laser-Lichts mit welchem das Mess-Zielobjekt bestrahlt wird und (ii) eine zweite Licht-Empfangs-Vorrichtung (12), welche das zweite empfangene Lichtsignal erlangt durch Empfangen des Laser-Lichts mit welchem das Mess-Zielobjekt bestrahlt wird,
ein Licht-empfangender Bereich der ersten
Licht-Empfangs-Vorrichtung (22) größer ist
als ein Licht-empfangender Bereich der
zweiten Licht-Empfangs-Vorrichtung (12).

2. Die Fluid-Erfassungs-Vorrichtung (1) gemäß Anspruch 1, wobei

die Bestrahlungs-Vorrichtung aufweist: (i) eine erste Bestrahlungs-Vorrichtung (41), welche das Mess-Zielobjekt mit einem Mess-Licht bestrahlt, welches ein Licht ist, dessen Art von der Art des Laser-Lichts verschieden ist; und (ii) eine zweite Bestrahlungs-Vorrichtung (11), welches das Mess-Zielobjekt mit dem Laser-Licht (LB) bestrahlt,
die Licht-Empfangs-Vorrichtung (12) (i) das erste empfangene Lichtsignal erlangt durch Empfangen des Mess-Lichts mit welchem das Mess-Zielobjekt bestrahlt wird und (ii) das zweite empfangene Lichtsignal zu erlangen durch Empfangen des Laser-Lichts mit welchem das Mess-Zielobjekt bestrahlt wird,
die erste Ermittlungs-Vorrichtung (143) eingerichtet ist, die Konzentration (N) anhand des ersten empfangenen Lichtsignals zu ermitteln,
die zweite Ermittlungs-Vorrichtung (146) eingerichtet ist, die Flussrate (Q) und/oder die Strömungsgeschwindigkeit (v) zu ermitteln anhand (i) des zweiten empfangene Lichtsignals und (ii) der Konzentration, welche die erste Ermittlungs-Vorrichtung ermittelt.

3. Die Fluid-Erfassungs-Vorrichtung (1) gemäß Anspruch 1, wobei

die erste Ermittlungs-Vorrichtung (143) eingerichtet ist, die Konzentration (N) anhand des ersten empfangenen Lichtsignals zu ermitteln,
die zweite Ermittlungs-Vorrichtung (146) eingerichtet ist, die Flussrate (Q) und/oder die Strömungsgeschwindigkeit (v) zu ermitteln anhand (i) des zweiten empfangene Lichtsignals und (ii) der Konzentration, welche die erste Ermittlungs-Vorrichtung ermittelt.

4. Die Fluid-Erfassungs-Vorrichtung (1) gemäß Anspruch 2, wobei

die Licht-Empfangs-Vorrichtung aufweist: (i) eine erste Licht-Empfangs-Vorrichtung (22), welche das erste empfangene Lichtsignal erlangt durch Empfangen des Mess-Lichts mit welchem das Mess-Zielobjekt bestrahlt wird und (ii) eine zweite Licht-Empfangs-Vorrichtung (12), welche das zweite empfangene Lichtsignal erlangt durch Empfangen des Laser-Lichts mit welchem das Mess-Zielobjekt bestrahlt wird,
die erste Ermittlungs-Vorrichtung (143) eingerichtet ist, die Konzentration (N) anhand des ersten empfangenen Lichtsignals zu ermitteln,
die zweite Ermittlungs-Vorrichtung (146) eingerichtet ist, die Flussrate (Q) und/oder die Strömungsgeschwindigkeit (v) zu ermitteln anhand (i) des zweiten empfangene Lichtsignals und (ii) der Konzentration, welche die erste Ermittlungs-Vorrichtung ermittelt.

5. Die Fluid-Erfassungs-Vorrichtung (1) gemäß Anspruch 1, wobei
die zweite Ermittlungs-Vorrichtung (143) eingerichtet ist, die Flussrate (Q) und/oder die Strömungsgeschwindigkeit (v) auf der Basis von Korrelationsinformationen zu ermitteln, wobei die Korrelationsinformationen zumindest eine erste Korrelation zwischen der Variation der Frequenz und der Flussrate und/oder der Strömungsgeschwindigkeit für den Fall, dass die Konzentration (N) ein erster Wert ist, und eine zweite Korrelation zwischen der Variation der Frequenz und der Flussrate und/oder der Strömungsgeschwindigkeit für den Fall, dass die Konzentration ein zweiter Wert ist, definieren.

6. Die Fluid-Erfassungs-Vorrichtung (1) gemäß Anspruch 1, wobei
die zweite Ermittlungs-Vorrichtung (146) eingerichtet ist, die Flussrate (Q) und/oder die Strömungsgeschwindigkeit (v) auf der Basis von Korrelationsinformationen zu ermitteln, wobei die Korrelationsinformationen zumindest eine erste Korrelation zwischen einer Durchschnittsfrequenz (Fm), welche durch Analysieren des zweiten empfangenen Lichtsignals erlangt werden kann, und der Flussrate und/oder der Strömungsgeschwindigkeit für den Fall, dass die Konzentration (N) ein erster Wert ist, und eine zweite Korrelation zwischen der Durchschnittsfrequenz und der Flussrate und/oder der Strömungsgeschwindigkeit für den Fall, dass die Konzentration ein zweiter Wert ist, definieren.

7. Die Fluid-Erfassungs-Vorrichtung (1) gemäß Anspruch 6, wobei
die Korrelationsinformationen die erste Korrelation und die zweite Korrelation definieren, welche repräsentieren, dass die Flussrate (Q) und/oder die Strömungsgeschwindigkeit (v), welche ermittelt wird, wenn die Konzentration (N) der erste Wert ist, kleiner ist als die Flussrate und/oder die Strömungsgeschwindigkeit, welche ermittelt wird, wenn die Konzentration der zweite Wert ist, der größer ist als der erste Wert, unter einer solchen Bedingung, dass sich die Durchschnittsfrequenz (Fm) nicht verändert.

8. Die Fluid-Erfassungs-Vorrichtung (1) gemäß Anspruch 1, wobei

die zweite Ermittlungs-Vorrichtung (146) eingerichtet ist, die Flussrate (Q) und/oder die Strömungsgeschwindigkeit (v) in einem zweiten Zyklus zu ermitteln,
die erste Ermittlungs-Vorrichtung (143) eingerichtet ist, die Konzentration (N) in einem ersten Zyklus, der länger ist als der zweite Zyklus, zu ermitteln.

9. Die Fluid-Erfassungs-Vorrichtung (1) gemäß Anspruch 1, wobei
das Fluid ein Blut eines lebenden Körper ist, wobei ein Abschnitt des lebenden Körpers das Mess-Zielobjekt ist.

10. Die Fluid-Erfassungs-Vorrichtung (1) gemäß Anspruch 1, wobei

das erste empfangene Lichtsignal aus dem empfangenen Lichtsignal des das Mess-Zielobjekt (100) durchdringenden Laser-Lichts erzeugt ist,
das zweite empfangene Lichtsignal aus dem empfangenen Lichtsignal des von dem Mess-Zielobjekt (100) reflektierten Laser-Lichts erzeugt ist.

11. Ein Fluid-Erfassungs-Verfahren unter Verwendung einer Fluid-Erfassungs-Vorrichtung (1),
wobei die Fluid-Erfassungs-Vorrichtung aufweist:

eine Bestrahlungs-Vorrichtung (11), welche eingerichtet ist, ein Mess-Zielobjekt (100) zumindest mit einem Laser-Licht (LB) zu bestrahlen, wobei ein Fluid in einem Inneren des Mess-Zielobjekts fließt; und
eine Licht-Empfangs-Vorrichtung (12), welche eingerichtet ist, zumindest das Laser-Licht zu empfangen, mit welchem das Mess-Zielobjekt bestrahlt wird,
ein erster Ermittlungsvorgang, welcher eine Konzentration (N) des Fluids ermittelt anhand eines ersten empfangenen Lichtsignals basierend auf einer Signalintensität eines empfangenen Lichtsignals, welches die Licht-Empfangs-Vorrichtung empfängt; and
ein zweiter Ermittlungsvorgang, welcher eine Flussrate (Q) des Fluids und/oder eine Strömungsgeschwindigkeit (v) des Fluids ermittelt anhand (i) eines zweiten empfangenen Lichtsignals basierend auf einer Variation einer Frequenz des empfangenen Lichtsignals, welches durch eine Dopplerverschiebung des Laser-Lichts, mit welchem das Mess-Zielobjekt bestrahlt wird, verursacht wird, und (ii) der Konzentration, welche der erste Ermittlungsvorgang ermittelt,
wobei die Licht-Empfangs-Vorrichtung eine erste Fotodiode (121) und eine zweite Fotodiode (122) aufweist, von welchen jede eingerichtet ist, das Laser-Licht zu empfangen und das Laser-Licht in elektrischen Strom (IdtA, IdtB) umzuwandeln,
**dadurch gekennzeichnet, dass**:

die erste Fotodiode und die zweite Fotodiode derart in Reihe geschaltet sind, dass Knoten gleicher Polarität der ersten Fotodiode und der zweiten Fotodiode miteinander gekoppelt sind,
die Licht-Empfangs-Vorrichtung eingerichtet ist, als das zweite empfangene Lichtsignal einen elektrischen Differenzstrom (IdtC) zwischen dem von der ersten Fotodiode ausgegebenen elektrischen Strom (IdtA) und dem von der zweiten Fotodiode ausgegebenen elektrischen Strom (IdtB) auszugeben,
der zweite Ermittlungsvorgang das zweite empfangene Lichtsignal in ein Spannungssignal umwandelt, verstärkt und das Spannungssignal ausgibt,
die Signalintensität eine Signalintensität einer Signalkomponente des empfangenen Lichtsignals ist, de ssen Frequenz relativ niedrig ist; and
die Variation der Frequenz eine Variation einer Frequenz einer Signalkomponente des empfangenen Lichtsignals ist, dessen Frequenz relativ hoch ist,
die Licht-Empfangs-Vorrichtung aufweist: (i) eine erste Licht-Empfangs-Vorrichtung (22), welche das erste empfangene Lichtsignal erlangt durch Empfangen des Laser-Lichts mit welchem das Mess-Zielobjekt bestrahlt wird und (ii) eine zweite Licht-Empfangs-Vorrichtung (12), welche das zweite empfangene Lichtsignal erlangt durch Empfangen des Laser-Lichts mit welchem das Mess-Zielobjekt bestrahlt wird,
ein Licht-empfangender Bereich der ersten Licht-Empfangs-Vorrichtung (22) größer ist als ein Licht-empfangender Bereich der zweiten Licht-Empfangs-Vorrichtung (12).

## Revendications

1. Appareil d'évaluation de fluide (1), comprenant :

un dispositif d'irradiation (11) configuré pour irradier une cible de mesure (100) avec au moins une lumière laser (LB), un fluide s'écoulant à l'intérieur de la cible de mesure ; et
un dispositif de réception de lumière (12) configuré pour recevoir au moins la lumière laser avec laquelle la cible de mesure est irradiée ;

un premier dispositif de calcul (143) configuré pour calculer une concentration (N) du fluide à partir d'un premier signal lumineux reçu, sur la base d'une intensité de signal d'un signal lumineux reçu que le dispositif de réception de lumière reçoit ; et

un deuxième dispositif de calcul (146) configuré pour calculer un débit (Q) du fluide et/ou une vitesse d'écoulement (v) du fluide à partir (i) d'un deuxième signal lumineux reçu sur la base d'une variation d'une fréquence du signal lumineux reçu qui est causée par un décalage Doppler de la lumière laser avec laquelle la cible de mesure est irradiée et (ii) de la concentration que le premier dispositif de calcul calcule,

le dispositif de réception de lumière comprenant une première photodiode (121) et une deuxième photodiode (122), dont chacune est configurée pour recevoir la lumière laser et convertir la lumière laser en courant électrique (IdtA, IdtB),

**caractérisé en ce que** :

les première et deuxième photodiodes étant couplées en série de sorte que des noeuds de même polarité des première et deuxième photodiodes sont couplés l'un à l'autre,

le dispositif de réception de lumière étant configuré pour émettre, en tant que deuxième signal lumineux reçu, un courant électrique différentiel (IdtC) entre le courant électrique (IdtA) émis par la première photodiode et le courant électrique (IdtB) émis par la deuxième photodiode,

le deuxième dispositif de calcul comprenant un amplificateur (13) configuré pour amplifier et convertir le deuxième signal lumineux reçu en signal de tension et émettre le signal de tension,

l'intensité du signal est une intensité d'une composante de signal du signal lumineux reçu dont la fréquence est relativement faible ; et

la variation de la fréquence est une variation d'une fréquence d'une composante de signal du signal lumineux reçu dont la fréquence est relativement élevée,

le dispositif de réception de lumière comprend : (i) un premier dispositif de réception de lumière (22) qui obtient le premier signal lumineux reçu en recevant la lumière laser avec laquelle la cible de mesure est irradiée et (ii) un deuxième dispositif de réception de lumière (12) qui obtient le deuxième signal lumineux reçu en recevant la lumière laser avec laquelle la cible de mesure est irradiée,

une zone de réception de lumière du premier dispositif de réception de lumière (22) est plus grande qu'une zone de réception de lumière du deuxième dispositif de réception de lumière (12).

2. Appareil d'évaluation de fluide (1) selon la revendication 1, dans lequel

le dispositif d'irradiation comprend : (i) un premier dispositif d'irradiation (41) qui irradie la cible de mesure avec une lumière de mesure qui est une lumière dont le type est différent du type de la lumière laser ; et (ii) un deuxième dispositif d'irradiation (11) qui irradie la cible de mesure avec la lumière laser (LB),

le dispositif de réception de lumière (12) (i) obtient le premier signal lumineux reçu en recevant la lumière de mesure avec laquelle la cible de mesure est irradiée et (ii) obtient le deuxième signal lumineux reçu en recevant la lumière laser avec laquelle la cible de mesure est irradiée et

le premier dispositif de calcul (143) est configuré pour calculer la concentration (N) à partir du premier signal lumineux reçu,

le deuxième dispositif de calcul (146) est configuré pour calculer le débit (Q) et/ou la vitesse d'écoulement (v) à partir (i) du deuxième signal lumineux reçu et (ii) de la concentration que le premier dispositif de calcul calcule.

3. Appareil d'évaluation de fluide (1) selon la revendication 1, dans lequel

le premier dispositif de calcul (143) est configuré pour calculer la concentration (N) à partir du premier signal lumineux reçu,

le deuxième dispositif de calcul (146) est configuré pour calculer le débit (Q) et/ou la vitesse d'écoulement (v) à partir (i) du deuxième signal lumineux reçu et (ii) de la concentration que le premier dispositif de calcul calcule.

4. Appareil d'évaluation de fluide (1) selon la revendication 2, dans lequel

le dispositif de réception de lumière comprend : (i) un premier dispositif de réception de lumière (22) qui obtient le premier signal lumineux reçu en recevant la lumière de mesure avec laquelle la cible de mesure est irradiée et (ii) un deuxième dispositif de réception de lumière (12) qui obtient le deuxième signal lumineux reçu en recevant la lumière laser avec laquelle la cible de mesure est irradiée,

le premier dispositif de calcul (143) est configuré pour calculer la concentration (N) à partir du premier signal lumineux reçu,

le deuxième dispositif de calcul (146) est configuré pour calculer le débit (Q) et/ou la vitesse d'écoulement (v) à partir (i) du deuxième signal lumineux reçu et (ii) de la concentration que le premier dispositif de calcul calcule.

5. Appareil d'évaluation de fluide (1) selon la revendication 1, dans lequel
le deuxième dispositif de calcul (143) est configuré pour calculer le débit (Q) et/ou la vitesse d'écoulement (v) sur la base d'informations de corrélation, dans lequel les informations de corrélation définissent au moins une première corrélation entre la variation de la fréquence et le débit et/ou la vitesse d'écoulement dans le cas où la concentration (N) est une première valeur et une deuxième corrélation entre la variation de la fréquence et le débit et/ou la vitesse d'écoulement dans le cas où la concentration est une deuxième valeur.

6. Appareil d'évaluation de fluide (1) selon la revendication 1, dans lequel
le deuxième dispositif de calcul (146) est configuré pour calculer le débit (Q) et/ou la vitesse d'écoulement (v) sur la base d'informations de corrélation, dans lequel les informations de corrélation définissent au moins une première corrélation entre une fréquence moyenne (Fm) qui peut être obtenue en analysant le deuxième signal lumineux reçu et le débit et/ou la vitesse d'écoulement dans le cas où la concentration (N) est une première valeur et une deuxième corrélation entre la fréquence moyenne et le débit et/ou la vitesse d'écoulement dans le cas où la concentration est une deuxième valeur.

7. Appareil d'évaluation de fluide (1) selon la revendication 6, dans lequel
les informations de corrélation définissent les première et deuxième corrélations représentant que le débit (Q) et/ou la vitesse d'écoulement (v) qui est calculé lorsque la concentration (N) est la première valeur est plus petit que le débit et/ou la vitesse d'écoulement qui est calculé lorsque la concentration est la deuxième valeur qui est plus grande que la première valeur dans une condition telle que la fréquence moyenne (Fm) ne change pas.

8. Appareil d'évaluation de fluide (1) selon la revendication 1, dans lequel
le deuxième dispositif de calcul (146) est configuré pour calculer le débit (Q) et/ou la vitesse d'écoulement (v) avec un deuxième cycle,
le premier dispositif de calcul (143) est configuré pour calculer la concentration (N) avec un premier cycle qui est plus long que le deuxième cycle.

9. Appareil d'évaluation de fluide (1) selon la revendication 1, dans lequel
le fluide est le sang d'un corps vivant, une partie du corps vivant étant la cible de mesure.

10. Appareil d'évaluation de fluide (1) selon la revendication 1, dans lequel
le premier signal lumineux reçu est généré à partir du signal lumineux reçu de la lumière laser traversant la cible de mesure (100),
le deuxième signal lumineux reçu est généré à partir du signal lumineux reçu de la lumière laser réfléchie par la cible de mesure (100).

11. Procédé d'évaluation de fluide utilisant un appareil d'évaluation de fluide (1), l'appareil d'évaluation de fluide comprenant :
un dispositif d'irradiation (11) configuré pour irradier une cible de mesure (100) avec au moins une lumière laser (LB), un fluide s'écoulant à un intérieur de la cible de mesure ; et
un dispositif de réception de lumière (12) configuré pour recevoir au moins la lumière laser avec laquelle la cible de mesure est irradiée,
un premier processus de calcul qui calcule une concentration (N) du fluide à partir d'un premier signal lumineux reçu, sur la base d'une intensité de signal d'un signal lumineux que le dispositif de réception de lumière reçoit ; et
un deuxième processus de calcul qui calcule un débit (Q) du fluide et/ou une vitesse d'écoulement (v) du fluide à partir (i) d'un deuxième signal lumineux reçu sur la base d'une variation d'une fréquence du signal lumineux reçu qui est causée par un décalage Doppler de la lumière laser avec laquelle la cible de mesure est irradiée et (ii) de la concentration que le premier processus de calcul calcule,
le dispositif de réception de lumière comprenant une première photodiode (121) et une deuxième photodiode (122), dont chacune est configurée pour recevoir la lumière laser et convertir la lumière laser en courant électrique (IdtA, IdtB),
**caractérisé en ce que** :
les première et deuxième photodiodes étant couplées en série de sorte que des noeuds de même polarité des première et deuxième photodiodes sont couplés l'un à l'autre,
le dispositif de réception de lumière étant configuré pour émettre, en tant que deuxième signal lumineux reçu, un courant électrique différentiel (IdtC) entre le courant électrique (IdtA) émis à partir de la première photodiode et le courant électrique (IdtB) émis à partir de la deuxième photodiode,

le deuxième processus de calcul amplifiant et convertissant le deuxième signal lumineux reçu en signal de tension et émettant le signal de tension,

l'intensité de signal est une intensité de signal d'une composante de signal du signal lumineux reçu dont la fréquence est relativement faible ; et

la variation de la fréquence est une variation d'une fréquence d'une composante de signal du signal lumineux reçu dont la fréquence est relativement élevée,

le dispositif de réception de lumière comprend : (i) un premier dispositif de réception de lumière (22) qui obtient le premier signal lumineux reçu en recevant la lumière laser avec laquelle la cible de mesure est irradiée et (ii) un deuxième dispositif de réception de lumière (12) qui obtient le deuxième signal lumineux reçu en recevant la lumière laser avec laquelle la cible de mesure est irradiée,

une zone de réception de lumière du premier dispositif de réception de lumière (22) est plus grande qu'une zone de réception de lumière du deuxième dispositif de réception de lumière (12).

[FIG. 1]

[FIG. 2]

```
                        ┌──────────────┐
                        │    START     │
                        └──────┬───────┘
                               │
                        ┌──────▼────────────┐
                        │ IRRADIATE TUBE WITH│    S11
                        │    LASER LIGHT     │
                        └──────┬─────────────┘
                               │
                        ┌──────▼─────────────┐
                        │      OBTAIN         │    S12
                        │ RECEIVED LIGHT SIGNAL│
                        └──────┬─────────────┘
                               │
                        ┌──────▼─────────────┐
                        │      AMPLIFY        │    S13
                        │ RECEIVED LIGHT SIGNAL│
                        └──────┬─────────────┘
                               │
```

| OBTAIN LIGHT INTENSITY SIGNAL BY HIGH FREQUENCY CUT OF AMPLIFIED RECEIVED SIGNAL — S14 | OBTAIN BEAT SIGNAL BY LOW FREQUENCY CUT OF AMPLIFIED RECEIVED SIGNAL — S17 |
|---|---|
| A/D SAMPLING OF LIGHT INTENSITY SIGNAL — S15 | A/D SAMPLING OF BEAT SIGNAL — S18 |
| CALCULATE BLOOD FLOW CONCENTRATION FROM SAMPLE VALUE OF LIGHT INTENSITY SIGNAL — S16 | CALCULATING BLOOD FLOW RATE Q FROM SAMPLE VALUE OF BEAT SIGNAL AND BLOOD FLOW CONCENTRATION N — S19 |

```
                        ┌──────────────┐
                        │     END      │
                        └──────────────┘
```

[FIG. 3]

(a)

(b)

[FIG. 4]

[FIG. 5]

POWER P(f)

BLOOD FLOW CONCENTRATION N
= LARGE

AVERAGE FREQUENCY Fm

BLOOD FLOW CONCENTRATION N
= SMALL

PARALLEL
MOVE

FREQUENCY f

(a)

POWER P(f)

BLOOD FLOW CONCENTRATION N
= LARGE

AVERAGE FREQUENCIES Fm, THAT
SHOULD BE THE SAME, ARE DIFFERENT

BLOOD FLOW CONCENTRATION N
= SMALL

AVERAGE FREQUENCY Fm

AMPLIFIER
NOISE

FREQUENCY f

(b)

[FIG. 6]

[FIG. 7]

[FIG. 8]

LASER
LIGHT LB

100

11 — LASER ELEMENT

SCATTERED LIGHT

SCATTERED LIGHT

22 — LIGHT RECEIVING ELEMENT

LIGH RECEIVING ELEMENT — 12

23 — AMPLIFIER

AMPLIFIER — 13

2

141 — LPF

HPF — 144

142 — A/D CONVERTER

A/D CONVERTER — 145

143 — PROCESSOR

PROCESSOR — 146

14

PROCESSING CIRCUIT

BLOOD FLOW
CONCENTRATION N

BLOOD FLOW
RATE Q

[FIG. 9]

EP 2 837 327 B1

[FIG. 10]

MEASUREMENT LIGHT          100          LASER LIGHT LB

LED ELEMENT — 41          LASER ELEMENT — 11

SCATTERED LIGHT OF MEASUREMENT LIGHT          SCATTERED LIGHT OF LASER LIGHT          4

22 — LIGHT RECEIVING ELEMENT          LIGHT RECEIVING ELEMENT — 12

23 — AMPLIFIER          AMPLIFIER — 13

14

141 — LPF          HPF — 144

142 — A/D CONVERTER          A/D CONVERTER — 145

143 — PROCESSOR          PROCESSOR — 146

PROCESSING CIRCUIT

BLOOD FLOW CONCENTRATION N          BLOOD FLOW RATE Q

36

[FIG. 11]

SCATTERED LIGHT

121    122

IdtA    IdtB

12

IdtC    —IdtC

13

In+    Vref    In—    131

DIFFERENTIAL AMPLIFIER    133

132

Out—    Out+

In—    In+

AMPLIFIER    134

Out

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4590948 A
- WO 2011070357 A1
- WO 2011161799 A1

- JP 3313841 B
- JP 62126569 A
- JP HEI716490 B